# EUROPEAN PATENT APPLICATION

(11) **EP 1 174 150 A1**
(43) Date of publication of application: **23.01.2002**
(21) Application number: 00919153.7
(22) Date of filing: 21.04.2000
(51) Int. Cl.: A61K 45/00, A61K 31/4545, A61K 31/444, A61K 31/501, A61K 31/505, A61K 31/415, A61K 31/437, A61K 31/519, A61K 31/53, A61K 31/195, A61K 31/445, C07D 213/81, C07D 239/50, C07D 231/40, C07D 237/08, C07D 401/12, C07D 401/14, C07D 409/14, C07D 471/04, C07D 487/04, C07D 495/04

(54) **PREVENTIVES/REMEDIES FOR ANGIOSTENOSIS**

(30) Priority: 22.04.1999 JP 11477599
(71) Applicant: Mitsubishi Pharma Corporation, Osaka-shi, Osaka 541-0046 (JP)
(72) Inventor: KAI, Hisashi, Chikushino-shi, Fukuoka 818-0053 (JP); UEHATA, Masayoshi, Mitsubihi Pharma Corporation, Schuo-ku, Tokyo 103-8405 (JP)
(74) Representative: von Kreisler, Alek, Dipl.-Chem.
(86) International application number: JP0002635
(87) International publication number: WO0064477

(57) **Abstract**

An agent for the prophylaxis and treatment of vascular constriction is provided, which contains a compound having a Rho kinase inhibitory activity. In particular, a compound having a Rho kinase inhibitory activity, for example, (+)-trans-4-(1-aminoethyl)-1-(4-pyridylcarbamoyl)cyclohexane, suppresses regenerative intima proliferation after disorder of blood vessel and has various other actions. Therefore, it is useful as an agent for the prophylaxis and treatment of vascular constriction, specifically, an agent for the prophylaxis and treatment of vascular constriction induced by disorder of vascular wall, such as vascular restenosis that occurs after an operation of percutaneus transluminal coronary angioplasty, vascular restenosis that occurs after an operation of percutaneus transluminal angioplasty, vascular constriction that occurs after vascular reconstruction, such as DCA, operation of intravascular indwelling of stent and the like, and vascular constriction that occurs after organ transplantation.

## Description

### Technical Field

The present invention relates to an agent for the prophylaxis and treatment of vascular constriction. More specifically, the present invention relates to an agent for the prophylaxis and treatment of vascular constriction, which agent comprises a compound having a Rho kinase inhibitory activity as an active ingredient.

More specifically, the present invention relates to an agent for the prophylaxis and treatment of vascular constriction induced by disorders of vascular walls. For example, it relates to an agent for the prophylaxis and treatment of vascular restenosis that occurs after operation of percutaneus transluminal coronary angioplasty, vascular restenosis that occurs after operation of percutaneus transluminal angioplasty, vascular constriction that occurs after operation of vascular reconstruction, such as DCA, intravascular indwelling of stent etc., and vascular constriction that occurs after organ transplantation.

### Background Art

With the progression of accumulation of lipids on vascular walls, cell proliferation on intima, and accumulation of collagen and the like, arteriosclerosis advances to cause tylosis or obliteration of vascular walls. When such condition is left untreated, serious situation follows, such as angina pectoris, myocardial infarction, cerebral infarction and the like. In recent years, vascular reconstruction, such as percutaneus transluminal coronary angioplasty (hereinafter to be referred to as PTCA), percutaneus transluminal angioplasty (hereinafter to be referred to as PTA), DCA (directional coronary atherectomy) wherein tissue lesion of a stenotic region is selectively cut, intravascular indwelling of stent and the like, has spread and has been clinically applied to ischemic heart diseases, such as angina pectoris, myocardial infarction and the like.

However, the physical removal of stenotic region as mentioned above causes damages of vascular intima, which in turn causes proliferation and migration into subintima of vascular smooth muscle cells, sometimes resulting in tylosis of the treated part. Particularly, PTCA and PTA give damage to blood vessels by insertion of balloon catheters, and may result in an incident of restenosis in the damaged lesion in several months after operation. In the case of DCA and intravascular indwelling of stent, moreover, vascular constriction may occur as in the case of PTCA, thereby posing a serious problem of vascular constriction in the aforementioned vascular reconstruction.

The vascular constriction is known to also occur after organ transplantation of heart, liver, kidney, blood vessel and the like, and vascular constriction after transplantation of these organs has also become an issue.

At present, for the prophylaxis and treatment of such vascular constriction, the use of anticoagulants, antilipemic agents, angiotensin converting enzyme inhibitors and the like has been tried, but none of them showed sufficient effects. Thus, the development of an agent for the prophylaxis and treatment of these vascular constrictions has become an important goal in cardiovascular internal medicine.

While the pharmaceutical use of a compound having a Rho kinase inhibitory activity is disclosed in WO98/06433, and described to be widely useful as a therapeutic agent of hypertension, a therapeutic agent of angina pectoris, a cerebrovascular spasm suppressant, a therapeutic agent of asthma, a therapeutic agent of peripheral circulatory disturbance, a premature delivery preventive, a therapeutic agent of arterial sclerosis, an anticancer drug, an anti-inflammatory agent, an immunosuppressant, a therapeutic agent of autoimmune diseases, an anti-AIDS agent, a therapeutic agent of osteoporosis, a therapeutic agent of retinopathy, a cerebral function improver, a contraceptive drug, and a gastrointestinal tract infection preventive. On the other hand, WO98/06433 does not teach its usefulness for the prevention and treatment of vascular constriction, or a description to suggest such effect.

As a compound having a Rho kinase inhibitory activity, a compound of the formula (I) to be mentioned later has been reported (WO98/06433). Certain isoquinolinesulfonamide derivative and isoquinoline derivative are also reported to show a Rho kinase inhibitory activity (WO98/06433 and Naunyn-Schmiedeberg's Archives of Pharmacology 385(1) Suppl., R219, 1998).

The compound of formula (I) has been already known to be useful as an agent for the prophylaxis and treatment of disorders of circulatory organs such as coronary, cerebral, renal, peripheral artery and the like (e.g., a therapeutic agent of hypertension, a therapeutic agent of angina pectoris, a therapeutic agent of renal and peripheral circulation disorder, a suppressive agent of cerebrovascular contraction and the like), which is potent and long lasting, and also as a therapeutic agent of asthma (JP-A-62-89679, JP-A-3-218356, JP-A-4-273821, JP-A-5-194401, JP-A-6-41080, WO95/28387, JP-A-62-89679, JP-A-3-218356, JP-A-4-273821, JP-A-5-194401, JP-A-6-41080, WO95/28387 etc.).

The isoquinolinesulfonamide derivative described in the above-mentioned WO98/06433 is known to be effective as a vasodilating agent, a therapeutic agent of hypertension, a cerebral function improver, an anti-asthma agent, a heart protecting agent, a platelet aggregation inhibitor, a therapeutic agent of neurologic manifestation, an anti-inflammatory agent, an agent for the treatment and prevention of hyperviscosity syndrome, a therapeutic agent of glaucoma, a diminished tension agent, a motor paralysis improver of cerebral thorombosis, an agent for prevention and treatment of virus infection and transcriptional control factor inhibitor (JP-A-57-200366, JP-A-61-227581, JP-A-2-256617, JP-A-4-264030, JP-A-6-56668, JP-A-6-80569, JP-A-6-293643, JP-A-7-41424, JP-A-7-277979, WO97/23222, JP-A-9-227381, JP-A-10-45598 and JP-A-10-87491).

Moreover, the isoquinoline derivative described in the above-mentioned publication (Naunyn-Schmiedeberg's Archives of Pharmacology 385(1) Suppl., R219, 1998) is known to be useful as an agent for the prevention and treatment of brain tissue disorder due to vasospasm (WO97/28130).

However, these compounds having Rho kinase inhibitory activity are not disclosed to be useful for prophylaxis and treatment of vascular constriction, and there is no description suggestive of such usefulness.

### Disclosure of the Invention

The present invention aims at solving the above-mentioned problems and provides a novel agent for the prophylaxis and treatment of vascular constriction, which agent is superior in a prophylactic and therapeutic effect on vascular constriction.

The present inventors have conducted intensive studies and found that a compound having a Rho kinase inhibitory activity has an effect of suppressing the proliferation of endometrium regenerated after vascular damage and various other effects, and that it is useful for the prophylaxis and treatment of vascular constriction, which resulted in the completion of the present invention.

Accordingly, the present invention provides the following.
(1) An agent for the prophylaxis and treatment of vascular constriction, which comprises a compound having a Rho kinase inhibitory activity.
(2) The agent for the prophylaxis and treatment of vascular constriction of (1) above, wherein the compound having a Rho kinase inhibitory activity is an amide compound of the following formula (I) 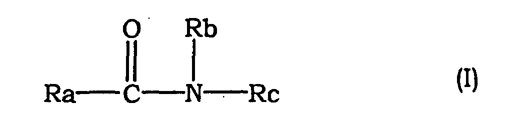 wherein
   - Ra: is a group of the formula 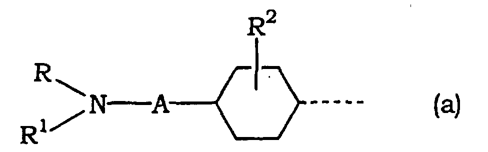 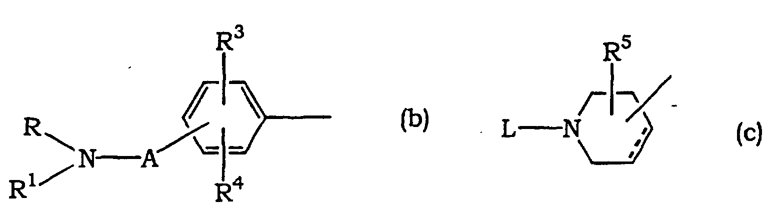
   in the formulas (a) and (b),
   - R: is hydrogen, alkyl, or cycloalkyl, cycloalkylalkyl, phenyl or aralkyl, which optionally has a substituent on the ring, or a group of the formula 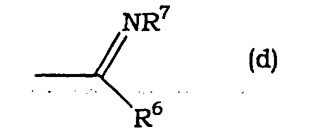 wherein R⁶ is hydrogen, alkyl or formula : -NR⁸R⁹ wherein R⁸ and R⁹ are the same or different and each is hydrogen, alkyl, aralkyl or phenyl, R⁷ is hydrogen, alkyl, aralkyl, phenyl, nitro or cyano, or R⁶ and R⁷ in combination show a group forming a heterocycle optionally having, in the ring, oxygen atom, sulfur atom or optionally substituted nitrogen atom,
   is hydrogen, alkyl, or cycloalkyl, cycloalkylalkyl, phenyl or aralkyl, which optionally has a substituent on the ring, or
   - R and R¹: in combination form, together with the adjacent nitrogen atom, a group forming a heterocycle optionally having, in the ring, oxygen atom, sulfur atom or optionally substituted nitrogen atom,
   - R²: is hydrogen or alkyl,
   - R³ and R⁴: are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and
   - A: is a group of the formula 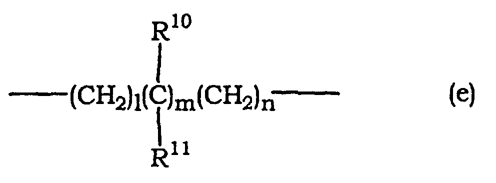 wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
   in the formula (c),
   - L: is hydrogen, alkyl, aminoalkyl, mono- or dialkylaminoalkyl, tetrahydrofurfuryl, carbamoylalkyl, phthalimidoalkyl, amidino or a group of the formula 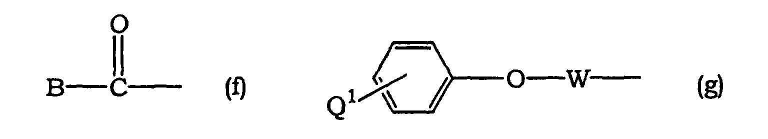 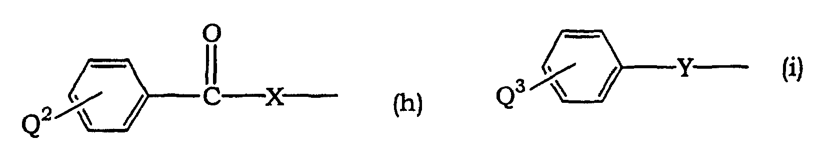 wherein B is hydrogen, alkyl, alkoxy, aralkyl, aralkyloxy, aminoalkyl, hydroxyalkyl, alkanoyloxyalkyl, alkoxycarbonylalkyl, α-aminobenzyl, furyl, pyridyl, phenyl, phenylamino, styryl or imidazopyridyl,
   Q¹ is hydrogen, halogen, hydroxy, aralkyloxy or thienylmethyl,
   W is alkylene,
   Q² is hydrogen, halogen, hydroxy or aralkyloxy,
   X is alkylene,
   Q³ is hydrogen, halogen, hydroxy, alkoxy, nitro, amino, 2,3-dihydrofuryl or 5-methyl-3-oxo-2,3,4,5-tetrahydropyridazin-6-yl;
   and Y is a single bond, alkylene or alkenylene, and
   in the formula (c),
   a broken line is a single bond or a double bond, and
   - R⁵: is hydrogen, hydroxy, alkoxy, alkoxycarbonyloxy,
   alkanoyloxy or aralkyloxycarbonyloxy;
   - Rb: is a hydrogen, an alkyl, an aralkyl, an aminoalkyl or a mono- or dialkylaminoalkyl; and
   - Rc: is an optionally substituted heterocycle containing nitrogen,
   an isomer thereof and/or a pharmaceutically acceptable acid addition salt thereof.
(3) The agent for the prophylaxis and treatment of vascular constriction of (1) or (2) above, wherein the compound having a Rho kinase inhibitory activity is an amide compound of the following formula (I') 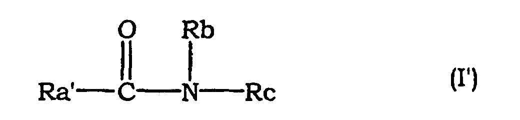 wherein
   - Ra': is a group of the formula 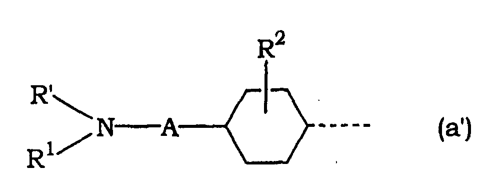 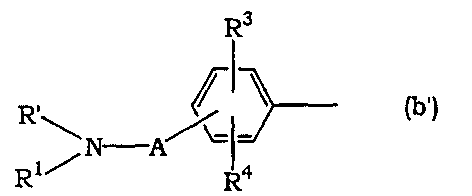 wherein
   - R'.: is hydrogen, alkyl, or cycloalkyl, cycloalkylalkyl, phenyl or aralkyl, which optionally has a substituent on the ring,
   - R¹: is hydrogen, alkyl, or cycloalkyl, cycloalkylalkyl, phenyl or aralkyl, which optionally has a substituent on the ring, or
   - R' and R¹: in combination form, together with the adjacent nitrogen atom, a group forming a heterocycle optionally having, in the ring, oxygen atom, sulfur atom or optionally substituted nitrogen atom,
   - R²: is hydrogen or alkyl,
   - R³ and R⁴: are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and
   - A: is a group of the formula 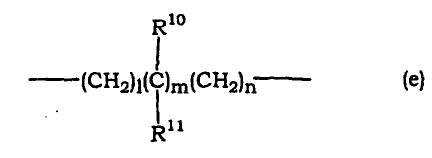 wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
   - Rb: is a hydrogen, an alkyl, an aralkyl, an aminoalkyl or a mono- or dialkylaminoalkyl; and
   - Rc: is an optionally substituted heterocycle containing nitrogen,
   an isomer thereof and/or a pharmaceutically acceptable acid addition salt thereof.
(4) The agent for the prophylaxis and treatment of vascular constriction of (1) above, wherein the compound having a Rho kinase inhibitory activity is a compound selected from the group consisting of (+)-trans-4-(1-aminoethyl)-1-(4-pyridylcarbamoyl)cyclohexane, (+)-trans-N-(1H-pyrrolo[2,3-b]pyridin-4-yl)-4-(1-aminoethyl)cyclohexanecarboxamide, (R)-(+)-N-(4-pyridyl)-4-(1-aminoethyl)benzamide and (R)-(+)-N-(1H-pyrrolo[2,3-b]pyridin-4-yl)-4-(1-aminoethyl)benzamide, and/or a pharmaceutically acceptable acid addition salt thereof.
(5) The agent for the prophylaxis and treatment of vascular constriction of (1) above, wherein the compound having a Rho kinase inhibitory activity is (+)-trans-4-(1-aminoethyl)-1-(4-pyridylcarbamoyl)cyclohexane and/or a pharmaceutically acceptable acid addition salt thereof.
(6) The agent for the prophylaxis and treatment of vascular constriction of any of (1) to (5) above, wherein the vascular constriction is induced by disorders of vascular walls.
(7) The agent for the prophylaxis and treatment of vascular constriction of any of (1) to (5) above, wherein the vascular constriction is vascular restenosis that occurs after an operation of percutaneus transluminal coronary angioplasty, vascular restenosis that occurs after an operation of percutaneus transluminal angioplasty, vascular constriction that occurs after vascular reconstruction, such as DCA, operation of intravascular indwelling of stent and the like, or vascular constriction that occurs after organ transplantation.
(8) A pharmaceutical composition for the prophylaxis and treatment of vascular constriction, which comprises a compound having a Rho kinase inhibitory activity and a pharmaceutically acceptable carrier.
(9) The pharmaceutical composition for the prophylaxis and treatment of vascular constriction of (8) above, wherein the compound having a Rho kinase inhibitory activity is an amide compound of the formula (I), an isomer thereof and/or a pharmaceutically acceptable acid addition salt thereof.
(10) The pharmaceutical composition for the prophylaxis and treatment of vascular constriction of (8) or (9) above, wherein the compound having a Rho kinase inhibitory activity is an amide compound of the formula (I'), an isomer thereof and/or a pharmaceutically acceptable acid addition salt thereof.
(11) The pharmaceutical composition for the prophylaxis and treatment of vascular constriction of (8) above, wherein the compound having a Rho kinase inhibitory activity is a compound selected from the group consisting of (+)-trans-4-(1-aminoethyl)-1-(4-pyridylcarbamoyl)cyclohexane, (+)-trans-N-(1H-pyrrolo[2,3-b]pyridin-4-yl)-4-(1-aminoethyl)cyclohexanecarboxamide, (R)-(+)-N-(4-pyridyl)-4-(1-aminoethyl)benzamide and (R)-(+)-N-(1H-pyrrolo[2,3-b]pyridin-4-yl)-4-(1-aminoethyl)benzamide, and/or a pharmaceutically acceptable acid addition salt thereof.
(12) The pharmaceutical composition for the prophylaxis and treatment of vascular constriction of (8) above, wherein the compound having a Rho kinase inhibitory activity is (+)-trans-4-(1-aminoethyl)-1-(4-pyridylcarbamoyl)cyclohexane and/or a pharmaceutically acceptable acid addition salt thereof.
(13) The pharmaceutical composition for the prophylaxis and treatment of vascular constriction of any of (8) to (12) above, wherein the vascular constriction is induced by a disorder of a vascular wall.
(14) The pharmaceutical composition for the prophylaxis and treatment of vascular constriction of any of (8) to (12) above, wherein the vascular constriction is vascular restenosis that occurs after an operation of percutaneus transluminal coronary angioplasty, vascular restenosis that occurs after an operation of percutaneus transluminal angioplasty, vascular constriction that occurs after vascular reconstruction, such as DCA, operation of intravascular indwelling of stent and the like, or vascular constriction that occurs after organ transplantation.
(15) A method of the prophylaxis and treatment of vascular constriction, which comprises administering an effective amount of a compound having a Rho kinase inhibitory activity to a patient.
(16) The method of the prophylaxis and treatment of vascular constriction of (15) above, wherein the compound having a Rho kinase inhibitory activity is an amide compound of the formula (I), an isomer thereof and/or a pharmaceutically acceptable acid addition salt thereof.
(17) The method of the prophylaxis and treatment of vascular constriction of (15) or (16) above, wherein the compound having a Rho kinase inhibitory activity is an amide compound of the formula (I'), an isomer thereof and/or a pharmaceutically acceptable acid addition salt thereof.
(18) The method of the prophylaxis and treatment of vascular constriction of (15) above, wherein the compound having a Rho kinase inhibitory activity is a compound selected from the group consisting of (+)-trans-4-(1-aminoethyl)-1-(4-pyridylcarbamoyl)cyclohexane, (+)-trans-N-(1H-pyrrolo[2,3-b]pyridin-4-yl)-4-(1-aminoethyl)cyclohexanecarboxamide, (R)-(+)-N-(4-pyridyl)-4-(1-aminoethyl)benzamide and (R)-(+)-N-(1H-pyrrolo[2,3-b]pyridin-4-yl)-4-(1-aminoethyl)benzamide, and/or a pharmaceutically acceptable acid addition salt thereof.
(19) The method of the prophylaxis and treatment of vascular constriction of (15) above, wherein the compound having a Rho kinase inhibitory activity is a (+)-trans-4-(1-aminoethyl)-1-(4-pyridylcarbamoyl)cyclohexane, and/or a pharmaceutically acceptable acid addition salt thereof.
(20) The method for the prophylaxis or treatment of vascular constriction of any of (15) to (19) above, wherein the vascular constriction is induced by a disorder of a vascular wall.
(21) The method for the prophylaxis or treatment of vascular constriction of any of (15) to (19) above, wherein the vascular constriction is vascular restenosis that occurs after an operation of percutaneus transluminal coronary angioplasty, vascular restenosis that occurs after an operation of percutaneus transluminal angioplasty, vascular constriction that occurs after vascular reconstruction, such as DCA, operation of intravascular indwelling of stent and the like, or vascular constriction that occurs after organ transplantation.
(22) Use of a compound having a Rho kinase inhibitory activity for the production of an agent for the prophylaxis and treatment of vascular constriction.
(23) The use of (22) above, wherein the compound having a Rho kinase inhibitory activity is an amide compound of the following formula (I), an isomer thereof and/or a pharmaceutically acceptable acid addition salt thereof.
(24) The use of (22) or (23) above, wherein the compound having a Rho kinase inhibitory activity is an amide compound of the following formula (I'), an isomer thereof and/or a pharmaceutically acceptable acid addition salt thereof.
(25) The use of (22) above, wherein the compound having a Rho kinase inhibitory activity is a compound selected from the group consisting of (+)-trans-4-(1-aminoethyl)-1-(4-pyridylcarbamoyl)-cyclohexane, (+)-trans-N-(1H-pyrrolo[2,3-b]pyridin-4-yl)-4-(1-aminoethyl)cyclohexanecarboxamide, (R)-(+)-N-(4-pyridyl)-4-(1-aminoethyl)benzamide and (R)-(+)-N-(1H-pyrrolo[2,3-b]pyridin-4-yl)-4-(1-aminoethyl)benzamide, and/or a pharmaceutically acceptable acid addition salt thereof.
(26) The use of (22) above, wherein the compound having a Rho kinase inhibitory activity is a (+)-trans-4-(1-aminoethyl)-1-(4-pyridylcarbamoyl)cyclohexane, and/or a pharmaceutically acceptable acid addition salt thereof.
(27) The use of any of (22) to (26) above, wherein the vascular constriction is induced by a disorder of a vascular wall.
(28) The use of any of (22) to (26) above, wherein the vascular constriction is vascular restenosis that occurs after an operation of percutaneus transluminal coronary angioplasty, vascular restenosis that occurs after an operation of percutaneus transluminal angioplasty, vascular constriction that occurs after vascular reconstruction, such as DCA, operation of intravascular indwelling of stent and the like, or vascular constriction that occurs after organ transplantation.
(29) A commercial package comprising a pharmaceutical composition for the prophylaxis and treatment of vascular constriction of any of (8) to (14) above, and a written matter associated therewith, the written matter stating that the pharmaceutical composition can or should be used for the prophylaxis and treatment of vascular constriction.

### Brief Description of the Drawings

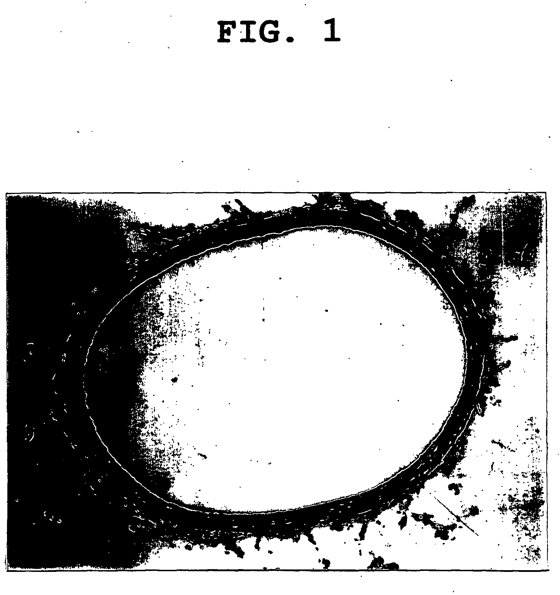

Fig. 1 is a microscopic photograph showing the results of one example from a sham group of Experimental Example 2 (effect on proliferation of intima after balloon injury of carotid artery in rats) of the present invention, wherein the extirpated left carotid artery was HE stained.

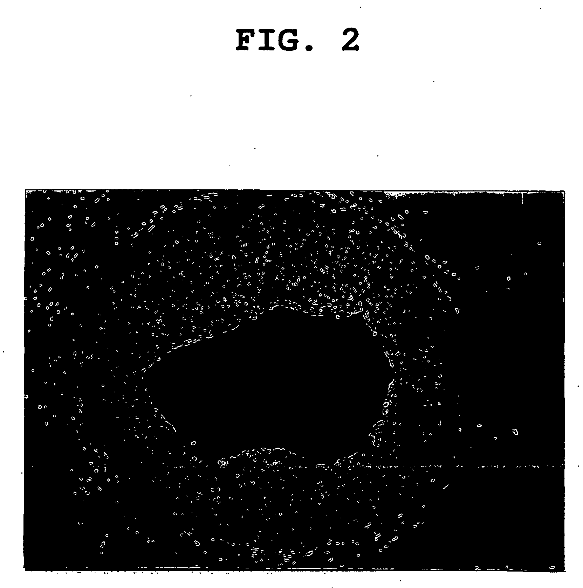

Fig. 2 is a microscopic photograph showing the results of one example from a control group (physiological saline administration group) of Experimental Example 2 (effect on proliferation of intima after balloon injury of carotid artery in rats) of the present invention at 14 days postoperation, wherein the extirpated left carotid artery was HE stained.

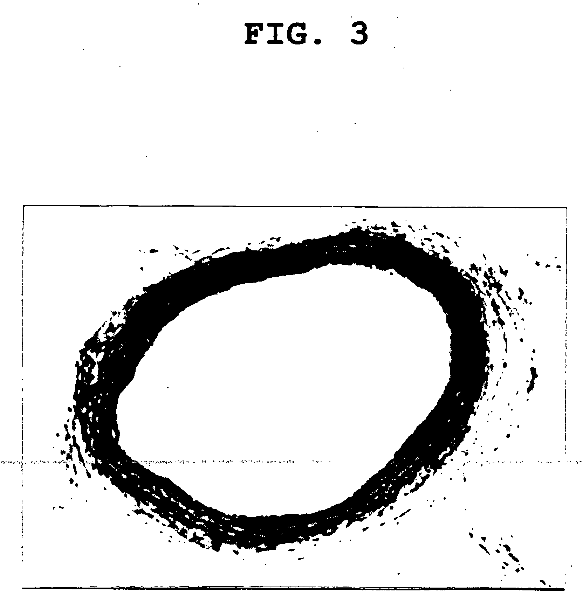

Fig. 3 is a microscopic photograph showing the results of one example from a test drug (Y-27632) administration group of Experimental Example 2 (effect on proliferation of intima after balloon injury of carotid artery in rats) of the present invention at 14 days postoperation, wherein the extirpated left carotid artery was HE stained.

### Detailed Description of the Invention

The vascular constriction in the present invention means the condition where a vascular wall shows tylosis or vascular lumen is occluded, which is induced by, for example, physical damage on the vascular wall. More specifically, examples thereof include vascular restenosis that occurs after an operation of percutaneus transluminal coronary angioplasty, vascular restenosis that occurs after an operation of percutaneus transluminal angioplasty, vascular constriction that occurs after vascular reconstruction, such as DCA, operation of intravascular indwelling of stent and the like, vascular constriction that occurs after organ transplantation and the like.

In the present invention, Rho kinase means serine/threonine kinase activated along with the activation of Rho. For example, ROKα (ROCKII: Leung, T. et al, J. Biol. Chem., 270, 29051-29054, 1995), p160 ROCK (ROKβ, ROCK-I: Ishizaki, T. et al, The EMBO J., 15(8), 1885-1893, 1996) and other proteins having a serine/threonine kinase activity are exemplified.

The compound having a Rho kinase inhibitory activity, which is used as an active ingredient in the present invention, may be any as long as it has a Rho kinase inhibitory activity. Specifically, there are mentioned amide compound, isoquinolinesulfonamide derivative and isoquinoline derivative described in the above-mentioned WO98/06433 and WO97/28130 [particularly Naunyn-Schmiedeberg's Archives of Pharmacology 385(1) Suppl., R219, 1998].

As the aforementioned amide compound, for example, a compound of the above-mentioned formula (I), particularly a compound of the formula (I'), are used. As the aforementioned isoquinolinesulfonic acid derivative, fasudil hydrochloride [hexahydro-1-(5-isoquinolinesulfonyl)-1H-1,4-diazepine] and the like are used. As the aforementioned isoquinoline derivative, hexahydro-1-[(4-methyl-5-isoquinolinyl)sulfonyl]-1H-1,4-diazepine dihydrochloride, (S)-(+)-hexahydro-2-methyl-1-[(4-methyl-5-isoquinolinyl) sulfonyl]-lH-1,4-diazepine hydrochloride, hexahydro-7-methyl-1-[(4-methyl-5-isoquinolinyl)sulfonyl]-1H-1,4-diazepine dihydrochloride, hexahydro-5-methyl-1-[(4-methyl-5-isoquinolinyl)sulfonyl]-1H-1,4-diazepine dihydrochloride, hexahydro-2-methyl-1-[(4-methyl-5-isoquinolinyl)sulfonyl]-1H-1,4-diazepine hydrochloride, (R)-(-)-hexahydro-2-methyl-1-[(4-methyl-5-isoquinolinyl)sulfonyl]-1H-1,4-diazepine hydrochloride, (R)-(+)-hexahydro-5-methyl-1-[(4-methyl-5-isoquinolinyl)sulfonyl]-1H-1,4-diazepine hydrochloride and the like are used.

Preferably, an amide compound of the formula (I), particularly preferably an amide compound of the formula (I'), is used.

In the present invention, one kind of a compound having a Rho kinase inhibitory activity may be used alone, or, where necessary, several kinds may be concurrently used.

In the present specification, each symbol of the formulas (I) and (I') is defined as follows.

Alkyl at R, R' and R¹ is linear or branched alkyl having 1 to 10 carbon atoms, which is exemplified by methyl, ethyl, propyl, isopropyl, butyl, isobutyl, sec-butyl, tert-butyl, pentyl, hexyl, heptyl, octyl, nonyl, decyl and the like, with preference given to alkyl having 1 to 4 carbon atoms.

Cycloalkyl at R, R' and R¹ has 3 to 7 carbon atoms and is exemplified by cyclopropyl, cyclobutyl, cyclopentyl, cyclohexyl, cycloheptyl and the like.

Cycloalkylalkyl at R, R' and R¹ is that wherein the cycloalkyl moiety is the above-mentioned cycloalkyl having 3 to 7 carbon atoms and the alkyl moiety is linear or branched alkyl having 1 to 6 carbon atoms (e.g., methyl, ethyl, propyl, isopropyl, butyl, pentyl, hexyl and the like), which is exemplified by cyclopropylmethyl, cyclobutylmethyl, cyclopentylmethyl, cyclohexylmethyl, cycloheptylmethyl, cyclopropylethyl, cyclopentylethyl, cyclohexylethyl, cycloheptylethyl, cyclopropylpropyl, cyclopentylpropyl, cyclohexylpropyl, cycloheptylpropyl, cyclopropylbutyl, cyclopentylbutyl, cyclohexylbutyl, cycloheptylbutyl, cyclopropylhexyl, cyclopentylhexyl, cyclohexylhexyl, cycloheptylhexyl and the like.

Aralkyl at R, R' and R¹ is that wherein alkyl moiety is alkyl having 1 to 4 carbon atoms and is exemplified by phenylalkyl such as benzyl, 1-phenylethyl, 2-phenylethyl, 3-phenylpropyl, 4-phenylbutyl and the like.

The substituent of "cycloalkyl, cycloalkylalkyl, phenyl or aralkyl, which optionally has a substituent on the ring", at R, R' and R¹ is halogen (e.g., chlorine, bromine, fluorine and iodine), alkyl (same as alkyl at R, R' and R¹), alkoxy (linear or branched alkoxy having 1 to 6 carbon atoms, such as methoxy, ethoxy, propoxy, isopropoxy, butoxy, isobutoxy, sec-butoxy, tert-butoxy, pentyloxy, hexyloxy and the like), aralkyl (same as aralkyl at R, R' and R¹) or haloalkyl (alkyl at R, R' and R¹ which is substituted by 1-5 halogen, and exemplified by fluoromethyl, difluoromethyl, trifluoromethyl, 2,2,2-trifluoroethyl, 2,2,3,3,3-pentafluoropropyl and the like), nitro, amino, cyano, azide and the like.

The group formed by R and R¹ or R' and R¹ in combination together with the adjacent nitrogen atom, which forms a heterocycle optionally having, in the ring, oxygen atom, sulfur atom or optionally substituted nitrogen atom is preferably a 5 or 6-membered ring and bonded ring thereof. Examples thereof include 1-pyrrolidinyl, piperidino, 1-piperazinyl, morpholino, thiomorpholino, 1-imidazolyl, 2,3-dihydrothiazol-3-yl and the like. The substituent of the optionally substituted nitrogen atom is exemplified by alkyl, aralkyl, haloalkyl and the like. As used herein, alkyl, aralkyl and haloalkyl are as defined for R, R' and R¹.

Alkyl at R² is as defined for R, R' and R¹.

Halogen, alkyl, alkoxy and aralkyl at R³ and R⁴ are as defined for R, R' and R¹.

Acyl at R³ and R⁴ is alkanoyl having 2 to 6 carbon atoms (e.g., acetyl, propionyl, butyryl, valeryl, pivaloyl and the like), benzoyl or phenylalkanoyl wherein the alkanoyl moiety has 2 to 4 carbon atoms (e.g., phenylacetyl, phenylpropionyl, phenylbutyryl and the like).

Alkylamino at R³ and R⁴ is that wherein the alkyl moiety is linear or branched alkyl having 1 to 6 carbon atoms. Examples thereof include methylamino, ethylamino, propylamino, isopropylamino, butylamino, isobutylamino, sec-butylamino, tert-butylamino, pentylamino, hexylamino and the like.

Acylamino at R³ and R⁴ is that wherein acyl moiety is alkanoyl having 2 to 6 carbon atoms, benzoyl, phenylalkanoyl wherein the alkanoyl moiety has 2 to 4 carbon atoms and the like, which is exemplified by acetylamino, propionylamino, butyrylamino, valerylamino, pivaloylamino, benzoylamino, phenylacetylamino, phenylpropionylamino, phenylbutyrylamino and the like.

Alkylthio at R³ and R⁴ is that wherein the alkyl moiety is linear or branched alkyl having 1 to 6 carbon atoms, which is exemplified by methylthio, ethylthio, propylthio, isopropylthio, butylthio, isobutylthio, sec-butylthio, tert-butylthio, pentylthio, hexylthio and the like.

Aralkyloxy at R³ and R⁴ is that wherein the alkyl moiety is alkyl having 1 to 4 carbon atoms, which is exemplified by benzyloxy, 1-phenylethyloxy, 2-phenylethyloxy, 3-phenylpropyloxy, 4-phenylbutyloxy and the like.

Aralkylthio at R³ and R⁴ is that wherein the alkyl moiety is alkyl having 1 to 4 carbon atoms, which is exemplified by benzylthio, 1-phenylethylthio, 2-phenylethylthio, 3-phenylpropylthio, 4-phenylbutylthio and the like.

Alkoxycarbonyl at R³ and R⁴ is that wherein the alkoxy moiety is linear or branched alkoxy having 1 to 6 carbon atoms, which is exemplified by methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl and the like.

Alkylcarbamoyl at R³ and R⁴ is carbamoyl mono- or di-substituted by alkyl having 1 to 4 carbon atoms, which is exemplified by methylcarbamoyl, dimethylcarbamoyl, ethylcarbamoyl, diethylcarbamoyl, propylcarbamoyl, dipropylcarbamoyl, butylcarbamoyl, dibutylcarbamoyl and the like.

Alkoxy at R⁵ is as defined for R, R' and R¹.

Alkoxycarbonyloxy at R⁵ is that wherein the alkoxy moiety is linear or branched alkoxy having 1 to 6 carbon atoms, which is exemplified by methoxycarbonyloxy, ethoxycarbonyloxy, propoxycarbonyloxy, isopropoxycarbonyloxy, butoxycarbonyloxy, isobutoxycarbonyloxy, sec-butoxycarbonyloxy, tert-butoxycarbonyloxy, pentyloxycarbonyloxy, hexyloxycarbonyloxy and the like.

Alkanoyloxy at R⁵ is that wherein the alkanoyl moiety is alkanoyl having 2 to 6 carbon atoms, which is exemplified by acetyloxy, propionyloxy, butyryloxy, valeryloxy, pivaloyloxy and the like.

Aralkyloxycarbonyloxy at R⁵ is that wherein the aralkyl moiety is aralkyl having C₁-C₄ alkyl, which is exemplified by benzyloxycarbonyloxy, 1-phenylethyloxycarbonyloxy, 2-phenylethyloxycarbonyloxy, 3-phenylpropyloxycarbonyloxy, 4-phenylbutyloxycarbonyloxy and the like.

Alkyl at R⁶ is as defined for R, R' and R¹; alkyl at R⁸ and R⁹ is as defined for R, R' and R¹; and aralkyl at R⁸ and R⁹ is as defined for R, R' and R¹.

Alkyl at R⁷ is as defined for R, R' and R¹ and aralkyl at R⁷ is as defined for R, R' and R¹.

The group formed by R⁶ and R⁷ in combination, which forms a heterocycle optionally having, in the ring, oxygen atom, sulfur atom or optionally substituted nitrogen atom, is imidazol-2-yl, thiazol-2-yl, oxazol-2-yl, imidazolin-2-yl, 3,4,5,6-tetrahydropyridin-2-yl, 3,4,5,6-tetrahydropyrimidin-2-yl, 1,3-oxazolin-2-yl, 1,3-thiazolin-2-yl or optionally substituted benzoimidazol-2-yl, benzothiazol-2-yl, benzoxazol-2-yl and the like having a substituent such as halogen, alkyl, alkoxy, haloalkyl, nitro, amino, phenyl, aralkyl and the like. As used herein, halogen, alkyl, alkoxy, haloalkyl and aralkyl are as defined for R, R' and R¹.

The substituent of the above-mentioned optionally substituted nitrogen atom is exemplified by alkyl, aralkyl, haloalkyl and the like. As used herein, alkyl, aralkyl and haloalkyl are as defined for R, R' and R¹.

Hydroxyalkyl at R¹⁰ and R¹¹ is linear or branched alkyl having 1 to 6 carbon atoms which is substituted by 1 to 3 hydroxy, which is exemplified by hydroxymethyl, 2-hydroxyethyl, 1-hydroxyethyl, 3-hydroxypropyl, 4-hydroxybutyl and the like.

Alkyl at R¹⁰ and R¹¹ is as defined for R, R' and R¹; haloalkyl and alkoxycarbonyl at R¹⁰ and R¹¹ are as defined for R, R' and R¹; aralkyl at R¹⁰ and R¹¹ is as defined for R, R' and R¹.

Cycloalkyl formed by R¹⁰ and R¹¹ in combination is the same as cycloalkyl at R, R' and R¹.

Alkyl at L is as defined for R, R' and R¹.

Aminoalky at L is a linear or branched alkyl having 1 to 6 carbon atoms, which is substituted by amino, which is exemplified by aminomethyl, 2-aminoethyl, 1-aminoethyl, 3-aminopropyl, 4-aminobutyl, 5-aminopentyl, 6-aminohexyl and the like.

Mono- or dialkylaminoalkyl at L is mono- or di-substituted aminoalkyl with alkyl having 1 to 4 carbon atoms, which is exemplified by methylaminomethyl, dimethylaminomethyl, ethylaminomethyl, diethylaminomethyl, propylaminomethyl, dipropylaminomethyl, butylaminomethyl, dibutylaminomethyl, 2-dimethylaminoethyl, 2-diethylaminoethyl and the like.

Carbamoylalkyl at L is linear or branched alkyl having 1 to 6 carbon atoms substituted by carbamoyl, which is exemplified by carbamoylmethyl, 2-carbamoylethyl, 1-carbamoylethyl, 3-carbamoylpropyl, 4-carbamoylbutyl, 5-carbamoylpentyl, 6-carbamoylhexyl and the like.

Phthalimidoalkyl at L is linear or branched alkyl having 1 to 6 carbon atoms, which is substituted by phthalimide. Examples thereof include phthalimidomethyl, 2-phthalimidoethyl, 1-phthalimidoethyl, 3-phthalimidopropyl, 4-phthalimidobutyl, 5-phthalimidopentyl, 6-phthalimidohexyl and the like.

Alkyl at B is as defined for R, R' and R¹.

Alkoxy at B is as defined for R, R' and R¹.

Aralkyl at B is as defined for R, R' and R¹.

Aralkyloxy at B is as defined for R³ and R⁴.

Aminoalkyl at B is as defined for L.

Hydroxyalkyl at B is as defined for R¹⁰ and R¹¹.

Alkanoyloxyalkyl at B is that wherein linear or branched alkyl having 1 to 6 carbon atoms is substituted by alkanoyloxy having alkanoyl moiety having 2 to 6 carbon atoms, which is exemplified by acetyloxymethyl, propionyloxymethyl, butyryloxymethyl, valeryloxymethyl, pivaloyloxymethyl, acetyloxyethyl, propionyloxyethyl, butyryloxyethyl, valeryloxyethyl, pivaloyloxyethyl and the like.

Alkoxycarbonylalkyl at B is that wherein linear or branched alkyl having 1 to 6 carbon atoms is substituted by alkoxycarbonyl having alkoxy moiety having 1 to 6 carbon atoms, which is exemplified by methoxycarbonylmethyl, ethoxycarbonylmethyl, propoxycarbonylmethyl, isopropoxycarbonylmethyl, butoxycarbonylmethyl, isobutoxycarbonylmethyl, sec-butoxycarbonylmethyl, tert-butoxycarbonylmethyl, pentyloxycarbonylmethyl, hexyloxycarbonylmethyl, methoxycarbonylethyl, ethoxycarbonylethyl, propoxycarbonylethyl, isopropoxycarbonylethyl, butoxycarbonylethyl, isobutoxycarbonylethyl, sec-butoxycarbonylethyl, tert-butoxycarbonylethyl, pentyloxycarbonylethyl, hexyloxycarbonylethyl and the like.

Halogen at Q¹, Q² and Q³ is as defined for R, R' and R¹.

Aralkyloxy at Q¹ and Q² is as defined for R³ and R⁴.

Alkoxy at Q³ is as defined for R, R' and R¹.

Alkylene at W, X and Y is linear or branched alkylene having 1 to 6 carbon atoms, which is exemplified by methylene, ethylene, trimethylene, propylene, tetramethylene, pentamethylene, hexamethylene and the like.

Alkenylene at Y is linear or branched alkenylene having 2 to 6 carbon atoms, which is exemplified by vinylene, propenylene, butenylene, pentenylene and the like.

Alkyl at Rb is as defined for R, R' and R¹.

Aralkyl at Rb is as defined for R, R' and R¹.

Aminoalkyl at Rb is as defined for L.

Mono- or dialkylaminoalkyl at Rb is as defined for L.

The heterocycle containing nitrogen at Rc is pyridine, pyrimidine, pyridazine, triazine, pyrazole, triazole and the like when it is a monocycle, and when it is a condensed ring, it is exemplified by pyrrolopyridine (e.g., 1H-pyrrolo[2,3-b]pyridine, 1H-pyrrolo[3,2-b]pyridine, 1H-pyrrolo[3,4-b]pyridine and the like), pyrazolopyridine (e.g., 1H-pyrazolo[3,4-b]pyridine, 1H-pyrazolo[4,3-b]pyridine and the like), imidazopyridine (e.g., 1H-imidazo[4,5-b]pyridine and the like), pyrrolopyrimidine (e.g., 1H-pyrrolo[2,3-d]pyrimidine, 1H-pyrrolo[3,2-d]pyrimidine, 1H-pyrrolo[3,4-d]pyrimidine and the like), pyrazolopyrimidine (e.g., 1H-pyrazolo[3,4-d]pyrimidine, pyrazolo[1,5-a]pyrimidine, 1H-pyrazolo[4,3-d]pyrimidine and the like), imidazopyrimidine (e.g., imidazo[1,2-a]pyrimidine, 1H-imidazo[4,5-d]pyrimidine and the like), pyrrolotriazine (e.g., pyrrolo[1,2-a]-1,3,5-triazine, pyrrolo[2,1-f]-1,2,4-triazine), pyrazolotriazine (e.g., pyrazolo[1,5-a]-1,3,5-triazine and the like), triazolopyridine (e.g., 1H-1,2,3-triazolo[4,5-b]pyridine and the like), triazolopyrimidine (e.g., 1,2,4-triazolo[1,5-a]pyrimidine, 1,2,4-triazolo[4,3-a]pyrimidine, 1H-1,2,3-triazolo[4,5-d]pyrimidine and the like), cinnoline, quinazoline, quinoline, pyridopyridazine (e.g., pyrido[2,3-c]pyridazine and the like), pyridopyrazine (e.g., pyrido[2,3-b]pyrazine and the like), pyridopyrimidine (e.g., pyrido[2,3-d]pyrimidine, pyrido[3,2-d]pyrimidine and the like), pyrimidopyrimidine (e.g., pyrimido[4,5-d]pyrimidine, pyrimido[5,4-d]pyrimidine and the like), pyrazinopyrimidine (e.g., pyrazino[2,3-d]pyrimidine and the like), naphthyridine (e.g., 1,8-naphthyridine and the like), tetrazolopyrimidine (e.g., tetrazolo[1,5-a]pyrimidine and the like), thienopyridine (e.g., thieno[2,3-b]pyridine and the like), thienopyrimidine (e.g., thieno[2,3-d]pyrimidine and the like), thiazolopyridine (e.g., thiazolo[4,5-b]pyridine, thiazolo[5,4-b]pyridine and the like), thiazolopyrimidine (e.g., thiazolo[4,5-d]pyrimidine, thiazolo[5,4-d]pyrimidine and the like), oxazolopyridine (e.g., oxazolo[4,5-b]pyridine, oxazolo[5,4-b]pyridine and the like), oxazolopyrimidine (e.g., oxazolo[4,5-d]pyrimidine, oxazolo[5,4-d]pyrimidine and the like), furopyridine (e.g., furo[2,3- b]pyridine, furo[3,2-b]pyridine and the like), furopyrimidine (e.g., furo[2,3-d]pyrimidine, furo[3,2-d]pyrimidine and the like), 2,3-dihydropyrrolopyridine (e.g., 2,3-dihydro-1H-pyrrolo[2,3-b]pyridine, 2,3-dihydro-1H-pyrrolo[3,2-b]pyridine and the like), 2,3-dihydropyrrolopyrimidine (e.g., 2,3-dihydro-1H-pyrrolo[2,3-d]pyrimidine, 2,3-dihydro-1H-pyrrolo[3,2-d]pyrimidine and the like), 5,6,7,8-tetrahydropyrido[2,3-d]pyrimidine, 5,6,7,8-tetrahydro-1,8-naphthyridine, 5,6,7,8-tetrahydroquinoline and the like. When these rings form a hydrogenated aromatic ring, the carbon atom in the ring may be carbonyl and includes, for example, 2,3-dihydro-2-oxopyrrolopyridine, 2,3-dihydro-2,3-dioxopyrrolopyridine, 7,8-dihydro-7-oxo-1,8-naphthyridine, 5,6,7,8-tetrahydro-7-oxo-1,8-naphthyridine and the like.

These rings may be substituted by a substituent such as halogen, alkyl, alkoxy, aralkyl, haloalkyl, nitro, amino, alkylamino, cyano, formyl, acyl, aminoalkyl, mono- or dialkylaminoalkyl, azide, carboxy, alkoxycarbonyl, carbamoyl, alkylcarbamoyl, alkoxyalkyl (e.g., methoxymethyl, methoxyethyl, methoxypropyl, ethoxymethyl, ethoxyethyl, ethoxypropyl and the like), optionally substituted hydrazino and the like.

As used herein, the substituent of the optionally substituted hydrazino includes alkyl, aralkyl, nitro, cyano and the like, wherein alkyl and aralkyl are as defined for R, R' and R¹ and exemplified by methylhydrazino, ethylhydrazino, benzylhydrazino and the like.

The compound of the formula (I) is exemplified by the following compounds.
(1) 4-(2-pyridylcarbamoyl)piperidine
(2) 1-benzyloxycarbonyl-4- (4-pyridylcarbamoyl)piperidine
(3) 1-benzoyl-4-(4-pyridylcarbamoyl)piperidine
(4) 1-propyl-4-(4-pyridylcarbamoyl)piperidine
(5) [3-(2-(2-thienylmethyl)phenoxy)-2-hydroxypropyl]-4-(4-pyridylcarbamoyl)piperidine
(6) 4-(4-pyridylcarbamoyl)piperidine
(7) 1-benzyl-4-(4-pyridylcarbamoyl)-1,2,5,6-tetrahydropyridine
(8) 3-(4-pyridylcarbamoyl)piperidine
(9) 1-benzyl-3-(4-pyridylcarbamoyl)piperidine
(10) 1-(2-(4-benzyloxyphenoxy)ethyl)-4-(N-(2-pyridyl)-N-benzylcarbamoyl)pyridine
(11) 1-formyl-4-(4-pyridylcarbamoyl)piperidine
(12) 4-(3-pyridylcarbamoyl)piperidine
(13) 1-isopropyl-4-(4-pyridylcarbamoyl)piperidine
(14) 1-methyl-4-(4-pyridylcarbamoyl)piperidine
(15) 1-hexyl-4-(4-pyridylcarbamoyl)piperidine
(16) 1-benzyl-4-(4-pyridylcarbamoyl)piperidine
(17) 1-(2-phenylethyl)-4-(4-pyridylcarbamoyl)piperidine
(18) 1-(2-(4-methoxyphenyl)ethyl)-4-(4-pyridylcarbamoyl)piperidine
(19) 1-(2-(4-methoxyphenyl)ethyl)-4-(2-pyridylcarbamoyl)piperidine
(20) 1-(2-(4-chlorophenyl)ethyl)-4-(4-pyridylcarbamoyl)piperidine
(21) 1-diphenylmethyl-4-(2-pyridylcarbamoyl)piperidine
(22) 1-[2-(4-(5-methyl-3-oxo-2,3,4,5-tetrahydropyridazin-6-yl)phenyl)ethyl]-4-(2-pyridylcarbamoyl)piperidine
(23) 1- (4- (4,5-dihydro-2-furyl)phenyl)-4-(4-pyridylcarbamoyl)piperidine
(24) 1-(2-nitrophenyl)-4-(4-pyridylcarbamoyl)piperidine
(25) 1-(2-aminophenyl)-4-(4-pyridylcarbamoyl)piperidine
(26) 1-nicotinoyl-4-(4-pyridylcarbamoyl)piperidine
(27) 1-isonicotinoyl-4-(4-pyridylcarbamoyl)piperidine
(28) 1- (3,4,5-trimethoxybenzoyl) -4- (4-pyridylcarbamoyl) piperidine
(29) 1-acetyl-4-(4-pyridylcarbamoyl)piperidine
(30) 1-(3-(4-fluorobenzoyl)propyl)-4-(4-pyridylcarbamoyl)piperidine
(31) 1-(3-(4-fluorobenzoyl)propyl)-4-(2-pyridylcarbamoyl)piperidine
(32) 1-(1-(4-hydroxybenzoyl)ethyl)-4-(2-pyridylcarbamoyl)piperidine
(33) 1-(1-(4-benzyloxybenzoyl)ethyl)-4-(2-pyridylcarbamoyl)piperidine
(34) 1-(2-(4-hydroxyphenoxy)ethyl)-4-(2-pyridylcarbamoyl)piperidine
(35) 1-(4-(4-fluorophenyl)-4-hydroxybutyl)-4-(4-pyridylcarbamoyl)piperidine
(36) 1-(1-methyl-2-(4-hydroxyphenyl)-2-hydroxyethyl)-4-(2-pyridylcarbamoyl)piperidine
(37) 1-cinnamyl-4-(2-pyridylcarbamoyl)piperidine
(38) 1-(2-hydroxy-3-phenoxypropyl)-4-(4-pyridylcarbamoyl)piperidine
(39) 1-(2-hydroxy-3-phenoxypropyl)-4-(3-pyridylcarbamoyl)piperidine
(40) 1-(2-hydroxy-3-phenoxypropyl)-4-(2-pyridylcarbamoyl)piperidine
(41) 1-(2-phenylethyl)-4-[N-(2-pyridyl)-N-(2-(N,N-dimethylamino)ethyl)carbamoyl]piperidine
(42) 1-benzyloxycarbonyl-4-(2-pyridylcarbamoyl)piperidine
(43) 1-(3-chlorophenyl)carbamoyl-4-(4-pyridylcarbamoyl)piperidine
(44) 1-[N-(2-pyridyl)-N-(2-(N,N-dimethylamino)ethyl)carbamoyl]piperidine
(45) 1-methyl-4-(4-pyridylcarbamoyl)-1,2,5,6-tetrahydropyridine
(46) 1-nicotinoyl-3-(4-pyridylcarbamoyl)piperidine
(47) 1-[2-(4-fluorobenzoyl)ethyl]-4-(4-pyridylcarbamoyl)piperidine
(48) 1-(6-chloro-2-methylimidazo[1,2-a]pyridine-3-carbonyl)-4-(4-pyridylcarbamoyl)piperidine
(49) 1-(4-nitrobenzyl)-4-(4-pyridylcarbamoyl)piperidine
(50) 1-hexyl-4-(4-pyridylcarbamoyl)piperidine
(51) 1-benzyloxycarbonyl-4-(2-chloro-4-pyridylcarbamoyl)piperidine
(52) 4-(2-chloro-4-pyridylcarbamoyl)piperidine
(53) 1-(2-chloronicotinoyl)-4-(4-pyridylcarbamoyl)piperidine
(54) 3-(2-chloro-4-pyridylcarbamoyl)piperidine
(55) 1-(4-phthalimidobutyl)-4-(4-pyridylcarbamoyl)piperidine
(56) 1-(3,5-di-tert-butyl-4-hydroxycinnamoyl)-4-(4-pyridylcarbamoyl)piperidine
(57) 1-carbamoylmethyl-4-(4-pyridylcarbamoyl)piperidine
(58) 1-benzyloxycarbonyl-4-(5-nitro-2-pyridylcarbamoyl)piperidine
(59) 4-(5-nitro-2-pyridylcarbamoyl)piperidine
(60) trans-4-benzyloxycarboxamidomethyl-1-(4-pyridylcarbamoyl)cyclohexane
(61) trans-4-aminomethyl-1-(4-pyridylcarbamoyl)cyclohexane
(62) trans-4-formamidomethyl-1-(4-pyridylcarbamoyl)cyclohexane
(63) trans-4-dimethylaminomethyl-1-(4-pyridylcarbamoyl)cyclohexane
(64) N-benzylidene-trans-(4-pyridylcarbamoyl)cyclohexylmethylamine
(65) trans-4-benzylaminomethyl-1-(4-pyridylcarbamoyl)cyclohexane
(66) trans-4-isopropylaminomethyl-1-(4-pyridylcarbamoyl)cyclohexane
(67) trans-4-nicotinoylaminomethyl-1-(4-pyridylcarbamoyl)cyclohexane
(68) trans-4-cyclohexylaminomethyl-1-(4-pyridylcarbamoyl)cyclohexane
(69) trans-4-benzyloxycarboxamide-1-(4-pyridylcarbamoyl)cyclohexane
(70) trans-4-amino-1-(4-pyridylcarbamoyl)cyclohexane
(71) trans-4-(1-aminoethyl)-1-(4-pyridylcarbamoyl)cyclohexane
(72) trans-4-aminomethyl-cis-2-methyl-1-(4-pyridylcarbamoyl)cyclohexane
(73) (+)-trans-4-(1-benzyloxycarboxamidopropyl)-1-cyclohexanecarboxylic acid
(74) (+)-trans-4-(1-benzyloxycarboxamidopropyl)-1-(4-pyridylcarbamoyl)cyclohexane
(75) (-)-trans-4-(1-benzyloxycarboxamidpropyl)-1-(4-pyridylcarbamoyl)cyclohexane
(76) (+)-trans-4- (1-aminopropyl) -1- (4-pyridylcarbamoyl)cyclohexane
(77) (-)-trans-4-(1-aminopropyl)-1-(4-pyridylcarbamoyl)cyclohexane
(78) (-)-trans-4-(1-benzyloxycarboxamidoethyl)-1-(4-pyridylcarbamoyl)cyclohexane
(79) (+)-trans-4-(1-benzyloxycarboxamidoethyl)-1-(4-pyridylcarbamoyl)cyclohexane
(80) (+)-trans-4-(1-aminoethyl)-1-(4-pyridylcarbamoyl)cyclohexane
(81) (-)-trans-4-(1-aminoethyl)-1-(4-pyridylcarbamoyl)cyclohexane
(82) trans-4-(4-chlorobenzoyl)aminomethyl-1-(4-pyridylcarbamoyl)cyclohexane
(83) trans-4-aminomethyl-1-(2-pyridylcarbamoyl)cyclohexane
(84) trans-4-benzyloxycarboxamidomethyl-1-(2-pyridylcarbamoyl)cyclohexane
(85) trans-4-methylaminomethyl-1-(4-pyridylcarbamoyl)cyclohexane
(86) trans-4-(N-benzyl-N-methylamino)methyl-1-(4-pyridylcarbamoyl)cyclohexane
(87) trans-4-aminomethyl-1-(3-pyridylcarbamoyl)cyclohexane
(88) trans-4-aminomethyl-1-[(3-hydroxy-2-pyridyl)carbamoyl]cyclohexane
(89) trans-4-benzyloxycarboxamidomethyl-1-(3-pyridylcarbamoyl)cyclohexane
(90) trans-4-benzyloxycarboxamidomethyl-1-[(3-benzyloxy-2-pyridyl)carbamoyl]cyclohexane
(91) trans-4-phthalimidomethyl-1-(4-pyridylcarbamoyl) cyclohexane
(92) trans-4-benzyloxycarboxamidomethyl-1-(3-methyl-4-pyridylcarbamoyl)cyclohexane
(93) trans-4-aminomethyl-1-(3-methyl-4-pyridylcarbamoyl)cyclohexane
(94) 4-(trans-4-benzyloxycarboxamidomethylcyclohexylcarbonyl)amino-2,6-dimethylpyridine-N-oxide
(95) 4-(trans-4-aminomethylcyclohexylcarbonyl)amino-2,6-dimethylpyridine-N-oxide
(96) trans-4-aminomethyl-1-(2-methyl-4-pyridylcarbamoyl)cyclohexane
(97) trans-4-(1-benzyloxycarboxamidoethyl)-1-(4-pyridylcarbamoyl)cyclohexane
(98) trans-4-(1-amino-1-methylethyl) -1- (4-pyridylcarbamoyl)cyclohexane
(99) trans-4-(2-aminoethyl)-1-(4-pyridylcarbamoyl)cyclohexane
(100) trans-4-(2-amino-1-methylethyl)-1-(4-pyridylcarbamoyl)cyclohexane
(101) trans-4-(1-aminopropyl)-1-(4-pyridylcarbamoyl)cyclohexane
(102) trans-4-aminomethyl-trans-1-methyl-1-(4-pyridylcarbamoyl)cyclohexane
(103) trans-4-benzylaminomethyl-cis-2-methyl-1-(4-pyridylcarbamoyl)cyclohexane
(104) trans-4-(1-benzyloxycarboxamide-1-methylethyl)-1-(4-pyridylcarbamoyl)cyclohexane
(105) trans-4-benzyloxycarboxamidomethyl-1-(N-methyl-4-pyridylcarbamoyl)cyclohexane
(106) trans-4-(1-acetamide-1-methylethyl)-1-(4-pyridylcarbamoyl)cyclohexane
(107) trans-N-(6-amino-4-pyrimidyl)-4-aminomethylcyclohexanecarboxamide
(108) trans-N-(1H-pyrrolo[2,3-b]pyridin-4-yl)-4-aminomethylcyclohexanecarboxamide
(109) (+)-trans-N-(1H-pyrrolo[2,3-b]pyridin-4-yl)-4-(1-aminoethyl)cyclohexanecarboxamide
(110) trans-N-(1H-pyrrolo[2,3-b]pyridin-4-yl)-4-(1-amino-1-methylethyl)cyclohexanecarboxamide
(111) trans-N-(1H-pyrazolo[3,4-b]pyridin-4-yl)-4-aminomethylcyclohexanecarboxamide
(112) (+)-trans-N-(1H-pyrazolo[3,4-b]pyridin-4-yl)-4-(1-aminoethyl)cyclohexanecarboxamide
(113) trans-N-(1H-pyrazolo[3,4-b]pyridin-4-yl)-4-(1-amino-1-methylethyl)cyclohexanecarboxamide
(114) (+)-trans-N-(2-amino-4-pyridyl)-4-(1-aminoethyl)cyclohexanecarboxamide
(115) trans-N-(1H-pyrazolo[3,4-d]pyrimidin-4-yl)-4-aminomethylcyclohexanecarboxamide
(116) (+)-trans-N-(1H-pyrazolo[3,4-d]pyrimidin-4-yl)-4-(1-aminoethyl)cyclohexanecarboxamide
(117) trans-N-(1H-pyrazolo[3,4-d]pyrimidin-4-yl)-4-(1-amino-1-methylethyl)cyclohexanecarboxamide
(118) trans-N-(4-pyrimidinyl)-4-aminomethylcyclohexanecarboxamide
(119) trans-N-(3-amino-4-pyridyl)-4-aminomethylcyclohexanecarboxamide
(120) trans-N- (7H-imidazo[4,5-d]pyrimidin-6-yl)-4-aminomethylcyclohexanecarboxamide
(121) trans-N-(3H-1,2,3-triazolo[4,5-d]pyrimidin-7-yl)-4-aminomethylcyclohexanecarboxamide
(122) trans-N-(1-benzyl-1H-pyrazolo[3,4-b]pyridin-4-yl)-4-aminomethylcyclohexanecarboxamide
(123) trans-N-(1H-5-pyrazolyl)-4-aminomethylcyclohexanecarboxamide
(124) trans-N-(1H-pyrazolo[3,4-b]pyridin-4-yl)-4-aminomethylcyclohexanecarboxamide
(125) trans-N-(4-pyridazinyl)-4-aminomethylcyclohexanecarboxamide
(126) trans-N-(7H-pyrrolo[2,3-d]pyrimidin-4-yl)-4-aminomethylcyclohexanecarboxamide
(127) trans-N-(2-amino-4-pyridyl)-4-aminomethylcyclohexanecarboxamide
(128) trans-N-(thieno[2,3-d]pyrimidin-4-yl)-4-aminomethylcyclohexanecarboxamide
(129) trans-N-(5-methyl-1,2,4-triazolo[1,5-a]pyrimidin-7-yl)-4-aminomethylcyclohexanecarboxamide
(130) trans-N-(3-cyano-5-methylpyrazolo[1,5-a]pyrimidin-7-yl)-4-aminomethylcyclohexanecarboxamide
(131) trans-N-(1H-pyrazolo[3,4-b]pyridin-4-yl)-4-(1-amino-1-methylethyl)cyclohexanecarboxamide
(132) trans-N-(2-(1-pyrrolidinyl)-4-pyridyl)-4-aminomethylcyclohexanecarboxamide
(133) trans-N- (2,6-diamino-4-pyrimidyl) -4-aminomethylcyclohexanecarboxamide
(134) (+)-trans-N-(7-methyl-1,8-naphthyridin-4-yl)-4-(1-aminoethyl)cyclohexanecarboxamide
(135) trans-N-(1-benzyloxymethylpyrrolo[2,3-b]pyridin-4-yl)-4-aminomethylcyclohexanecarboxamide
(136) (+)-trans-N-(1-methylpyrrolo[2,3-b]pyridin-4-yl)-4-(1-aminoethyl)cyclohexanecarboxamide
(137) trans-N-benzyl-N-(2-benzylamino-4-pyridyl)-4-(1-amino-1-methylethyl)cyclohexanecarboxamide
(138) trans-N-(2-azide-4-pyridyl)-4-aminomethylcyclohexanecarboxamide
(139) trans-N-(2,3-dihydro-1H-pyrrolo[2,3-b]pyridin-4-yl)-4-aminomethylcyclohexanecarboxamide
(140) trans-N-(2,3-dihydro-1H-pyrrolo[2,3-b]pyridin-4-yl)-4-(1-amino-1-methylethyl)cyclohexanecarboxamide
(141-1) trans-N-(2-carboxy-4-pyridyl)-4-aminomethylcyclohexanecarboxamide
(141-2) (R)-(+)-trans-N-(3-bromo-1H-pyrrolo[2,3-b]pyridin-4-yl)-4-(1-aminoethyl)cyclohexanecarboxamide
(142) trans-N-(1H-pyrrolo[2,3-b]pyridin-4-yl)-4-guanidinomethylcyclohexanecarboxamide
(143) trans-N-(1H-pyrazolo[3,4-b]pyridin-4-yl)-4-guanidinomethylcyclohexanecarboxamide
(144) trans-N-(4-pyridyl)-4-guanidinomethylcyclohexanecarboxamide
(145) trans-N-(1-methylpyrrolo[2,3-b]pyridin-4-yl)-4-(guanidinomethyl)cyclohexanecarboxamide
(146) trans-N-(1H-pyrrolo[2,3-b]pyridin-4-yl)-4-(2-imidazolin-2-yl)aminomethylcyclohexanecarboxamide
(147) trans-N-(1-benzyloxymethylpyrrplo[2,3-b]pyridin-4-yl)-4-guanidinomethylcyclohexanecarboxamide
(148) trans-N-(2-amino-4-pyridyl)-4-guanidinomethylcyclohexanecarboxamide
(149) trans-N-(1-benzyloxymethyl-1H-pyrrolo[2,3-b]pyridin-4-yl)-4-(2-imidazolin-2-yl)aminomethylcyclohexanecarboxamide
(150) trans-N-(1H-pyrrolo[2,3-b]pyridin-4-yl)-4-(3-benzylguanidinomethyl)cyclohexanecarboxamide
(151) trans-N-(1H-pyrrolo[2,3-b]pyridin-4-yl)-4-(3-phenylguanidinomethyl)cyclohexanecarboxamide
(152) trans-N-(1H-pyrrolo[2,3-b]pyridin-4-yl)-4-(3-propylguanidinomethyl)cyclohexanecarboxamide
(153) trans-N-(1H-pyrrolo[2,3-b]pyridin-4-yl)-4-(3-octylguanidinomethyl)cyclohexanecarboxamide
(154) trans-N-(1-benzyloxymethylpyrrolo[2,3-b]pyridin-4-yl)-4-(2-benzyl-3-ethylguanidinomethyl)cyclohexanecarboxamide
(155) trans-N-(1H-pyrrolo[2,3-b]pyridin-4-yl)-4-(imidazol-2-yl)aminomethylcyclohexanecarboxamide
(156) trans-N-(1H-pyrrolo[2,3-b]pyridin-4-yl)-4-(thiazol-2-yl)aminomethylcyclohexanecarboxamide
(157) (R)-(+)-N-(4-pyridyl)-4-(1-aminoethyl)benzamide
(158) N-(4-pyridyl)-4-(1-amino-1-methylethyl)benzamide
(159) N-(4-pyridyl)-4-aminomethyl-2-benzyloxybenzamide
(160) N-(4-pyridyl)-4-aminomethyl-2-ethoxybenzamide
(161) (R) - (-)-N-(4-pyridyl)-4-(1-aminoethyl)-3-nitrobenzamide
(162) (R)-(-)-N-(4-pyridyl)-3-amino-4-(1-aminoethyl)benzamide
(163) (R) - (+)-N-(4-pyridyl)-4-(1-aminoethyl)-3-chlorobenzamide
(164) N-(4-pyridyl)-3-aminomethylbenzamide
(165) (R)-(+)-N-(1H-pyrrolo[2,3-b]pyridin-4-yl)-4-(1-aminoethyl)benzamide
(166) (R)-(+)-N-(1H-pyrazolo[3,4-b]pyridin-4-yl)-4-(1-aminoethyl)benzamide
(167) N-(1H-pyrazolo[3,4-b]pyridin-4-yl)-4-guanidinomethylbenzamide
(168) N-(4-pyridyl)-4-guanidinomethylbenzamide
(169) (R)-(+)-N-(4-pyridyl)-4-(1-aminoethyl)-3-fluorobenzamide
(170) N-(4-pyridyl)-4-aminomethylbenzamide
(171) N-(4-pyridyl)-4-aminomethyl-2-hydroxybenzamide
(172) N-(4-pyridyl)-4-(2-aminoethyl)benzamide
(173) N-(4-pyridyl)-4-aminomethyl-3-nitrobenzamide
(174) N-(4-pyridyl)-3-amino-4-aminomethylbenzamide
(175) (S)-(-)-N-(4-pyridyl)-4-(1-aminoethyl)benzamide
(176) (S)-(-)-N-(4-pyridyl)-2-(1-aminoethyl)benzamide
(177) (R)-(+)-N-(4-pyridyl)-4-(1-aminoethyl)-2-chlorobenzamide
(178) (R)-(+)-N-(1H-pyrrolo[2,3-b]pyridin-4-yl)-4-(1-(3-propylguanidino)ethyl)benzamide
(179) (R)-(-)-N-(1H-pyrrolo[2,3-b]pyridin-4-yl)-4-(1-aminoethyl)-3-azidebenzamide
(180) (R) - (+)-N-(4-pyridyl) -4- (1-aminoethyl)-2-nitrobenzamide
(181) (R)-(-)-N-(4-pyridyl)-4-(1-aminoethyl)-3-ethoxybenzamide
(182) (R)-(+)-N-(3-iodo-lH-pyrrolo[2,3-b]pyridin-4-yl)-4-(1-aminoethyl)benzamide
(183) (R)-(+)-N-(3-iodo-1H-pyrrolo[2,3-b]pyridin-4-yl)-4-(1-aminoethyl)-3-azidebenzamide
(184) (R)-(-)-N-(4-pyridyl)-4-(1-aminoethyl)-3-hydroxybenzamide
(185) N-(1H-pyrazolo[3,4-b]pyridin-4-yl)-4-guanidinomethyl-3-nitrobenzamide
(186) (R)-N-(1H-pyrazolo[3,4-b]pyridin-4-yl)-4-(1-guanidinoethyl)-3-nitrobenzamide
(187) (R)-N-(1H-pyrazolo[3,4-b]pyridin-4-yl)-4-(1-aminoethyl)-2-nitrobenzamide
(188) N-(1H-pyrazolo[3,4-b]pyridin-4-yl)-4-guanidinobenzamide
(189) (R)-N-(1H-pyrazolo[3,4-b]pyridin-4-yl)-4-(1-aminoethyl)-3-nitrobenzamide
(190) (R)-N-(1H-pyrazolo[3,4-b]pyridin-4-yl)-4-(1-guanidinoethyl)benzamide
(191) N-(1H-pyrazolo[3,4-b]pyridin-4-yl)-4-(1-amino-2-hydroxyethyl)benzamide
(192) N-(1H-pyrazolo[3,4-b]pyridin-4-yl)-4-aminomethyl-3-nitrobenzamide
(193) N-(1H-pyrrolo[2,3-b]pyridin-4-yl)-4-piperidinecarboxamide
(194) N-(1H-pyrazolo[3,4-b]pyridin-4-yl)-4-piperidinecarboxamide
(195) N-(1H-pyrazolo[3,4-b]pyridin-4-yl)-1-aminoacetyl-4-piperidinecarboxamide
(196) N-(1-methoxymethyl-1H-pyrazolo[3,4-b]pyridin-4-yl)-4-piperidinecarboxamide
(197) N-(2,3-dihydro-1H-pyrrolo[2,3-b]pyridin-4-yl)-4-piperidinecarboxamide
(198) N-(1H-pyrrolo[2,3-b]pyridin-4-yl)-1-(2-phenylethyl)-4-piperidinecarboxamide
(199) N-(1H-pyrrolo[2,3-b]pyridin-4-yl)-1-amidino-4-piperidinecarboxamide
(200) N-(1H-pyrrolo[2,3-b]pyridin-4-yl)-1-(3-phenylpropyl)-4-piperidinecarboxamide
(201) N-(1H-pyrrolo[2,3-b]pyridin-4-yl)-1-benzyl-4-piperidinecarboxamide
(202) N-(1H-pyrazolo[3,4-b]pyridin-4-yl)-1-(2-phenylethyl)-4-piperidinecarboxamide
(203) N-(1H-pyrazolo[3,4-b]pyridin-4-yl)-1-(3-phenylpropyl)-4-piperidinecarboxamide

Preferred are compounds (80), (109), (110), (112), (115), (142), (143), (144), (145), (153), (157), (163), (165), (166) and (179).

The compound having a Rho kinase inhibitory activity may be a pharmaceutically acceptable acid addition salt, wherein the acid is exemplified by inorganic acid such as hydrochloric acid, hydrobromic acid, sulfuric acid and the like, and organic acid such as methanesulfonic acid, fumaric acid, maleic acid, mandelic acid, citric acid, tartaric acid, salicylic acid and the like. A compound having a carboxyl group can be converted to a salt with a metal such as sodium, potassium, calcium, magnesium, aluminum and the like, a salt with an amino acid such as lysine and the like. Further, monohydrate, dihydrate, 1/2 hydrate, 1/3 hydrate, 1/4 hydrate, 2/3 hydrate, 3/2 hydrate and the like are encompassed in the present invention.

The compound of the formula (I) can be synthesized by a method described in, for example, JP-A-62-89679, JP-A-3-218356, JP-A-5-194401, JP-A-6-41080, WO95/28387, WO98/06433 and the like.

When the above-mentioned compound having a Rho kinase inhibitory activity has an optical isomer, its racemate or cis-trans isomers, all of them can be used in the present invention. These isomers can be isolated by a conventional method or can be produced using starting materials of the isomers.

A compound having a Rho kinase inhibitory activity, particularly, a compound of the formula (I), an isomer thereof and/or a pharmaceutically acceptable acid addition salt thereof have a preventive and therapeutic effect on vascular constriction in mammals inclusive of human, cow, horse, dog, mouse, rat and the like. Therefore, they can be used as an agent for the prophylaxis and treatment of various types of vascular constriction.

The agent for the prophylaxis and treatment of vascular constriction of the present invention is administered orally or parenterally.

For example, the compound having a Rho kinase inhibitory activity is mixed with a pharmaceutically acceptable carrier (e.g., excipient, binder, disintegrator, corrective, corrigent, emulsifier, diluent, solubilizer and the like) to give a pharmaceutical composition or a pharmaceutical preparation in the form of tablet, pill, powder, granule, capsule, troche, syrup, liquid, emulsion, suspension, injection (e.g., liquid, suspension and the like), suppository, inhalant, percutaneous absorber, eye drop, eye ointment and the like in the form suitable for oral or parenteral preparation.

When preparing a solid preparation, additives such as sucrose, lactose, cellulose sugar, D-mannitol, maltitol, dextran, starches, agar, arginates, chitins, chitosans, pectines, tragacanth gum, gum arabic, gelatins, collagens, casein, albumin, calcium phosphate, sorbitol, glycine, carboxymethylcellulose, polyvinylpyrrolidone, hydroxypropylcellulose, hydroxypropylmethylcellulose, glycerol, polyethyleneglycol, sodium hydrogencarbonate, magnesium stearate, talc and the like are used. Tablets can be applied with a typical coating, where necessary, to give sugar coated tablets, enteric tablets, film-coated tablets, two-layer tablets and multi-layer tablets.

When preparing a semi-solid preparation, animal and plant fats and oils (e.g., olive oil, corn oil, castor oil and the like), mineral fats and oils (e.g., petrolatum, white petrolatum, solid paraffin and the like), wax (e.g., jojoba oil, carnauba wax, bee wax and the like), partly or entirely synthesized glycerol fatty acid esters (e.g., lauric acid, myristic acid, palmitic acid and the like), and the like are used.

Examples of commercially available products of these include Witepsol (manufactured by Dynamitnovel Ltd.), Farmazol (NOF Corporation) and the like.

When preparing a liquid preparation, an additive, such as sodium chloride, glucose, sorbitol, glycerol, olive oil, propylene glycol, ethyl alcohol and the like, is used. When preparing an injection, a sterile aqueous solution such as physiological saline, isotonic solution, oil (e.g., sesame oil and soybean oil) and the like are used. Where necessary, a suitable suspending agent such as sodium carboxymethylcellulose, nonionic surfactant, solubilizer (e.g., benzyl benzoate and benzyl alcohol), and the like can be concurrently used. Moreover, when an eye drop is prepared, an aqueous liquid or solution is used, which is particularly a sterile injectable aqueous solution. The eye drop can appropriately contain various additives such as buffer (borate buffer, acetate buffer, carbonate buffer and the like are preferable for reducing irritation), isotonicity agent, solubilizer, preservative, thickener, chelating agent, pH adjusting agent (generally, pH is preferably adjusted to about 6 - 8.5) and aromatic.

The dose of the compound having a Rho kinase inhibitory activity, which is the active ingredient of these preparations, is 0.1 - 100 wt%, suitably 1 - 50 wt%, of the preparation. While the dose varies depending on the symptom, body weight, age and the like of patients, it is generally about 1 - 500 mg a day for an adult, which is preferably administered once to several times a day.

### Examples

The present invention is explained in detail by referring to formulation examples and pharmacological action. The present invention is not limited in any way by the examples.

The method for preparing the pharmaceutical preparation of the present invention is explained in the following by way of Formulation Examples.

The compound having a Rho kinase inhibitory activity to be used in the present invention is also conveniently referred to as the inventive compound.

| **Formalation Example 1:** Tablet | |
|---|---|
| Compound of the present invention | 10.0 mg |
| Lactose | 50.0 mg |
| Corn starch | 20.0 mg |
| Crystalline cellulose | 29.7 mg |
| Polyvinylpyrrolidone K30 | 5.0 mg |
| Talc | 5.0 mg |
| Magnesium stearate | 0.3 mg |
| | 120.0 mg |

The compound of the present invention, lactose, corn starch and crystalline cellulose were mixed, kneaded with polyvinylpyrrolidone K30 paste solution and passed through a 20-mesh sieve for granulation. After drying at 50°C for 2 hours, the granules were passed through a 24-mesh sieve, and talc and magnesium stearate were added. Using a φ7 mm punch, tablets weighing 120 mg per tablet were prepared.

| **Formulation Example 2 :** Capsules | |
|---|---|
| Compound of the present invention | 10.0 mg |
| Lactose | 70.0 mg |
| Corn starch | 35.0 mg |
| Polyvinylpyrrolidone K30 | 2.0 mg |
| Talc | 2.7 mg |
| Magnesium stearate | 0.3 mg |
| | 120.0 mg |

The compound of the present invention, lactose and corn starch were mixed, kneaded with polyvinylpyrrolidone K30 paste solution and passed through a 20-mesh sieve for granulation. After drying at 50°C for 2 hours, the granules were passed through a 24-mesh sieve and talc and magnesium stearate were added. The mixture was filled in hard capsules (No. 4) to give capsules weighing 120 mg.

The pharmacological action of the pharmaceutical agent of the present invention is explained in the following by referring to Experimental Examples.

In the following Experimental Examples, a compound having a Rho kinase inhibitory activity: (+)-trans-4-(1-aminoethyl)-1-(4-pyridylcarbamoyl)cyclohexane 2HCl·1H₂O (hereinafter Y-27632) was used. Y-27632 was dissolved and diluted in physiological saline to achieve a predetermined concentration.

### Experimental Example 1: Effect on proliferation ability and migration ability of vascular smooth muscle cells (VSMC) derived from WKY rat aortic tunica media

### (1) Effect on proliferation ability of vascular smooth muscle cells (VSMC) derived from WKY rat aortic tunica media (method)

VSMC were separated and cultured according to the method of Yamamoto et al. (Yamamoto H, et al., Br. J. Exp. Pathol. 64; 156-165, 1983). The proliferation ability of VSMC was determined according to the method of Berk et al. [[³H] thymidine uptake method; Berk BC, et al., J. Cell Physiol. 137; 391-401, 1988]. A DMEM medium containing 10% serum was changed every two days and the cells were cultured on a dish. The cells after 5 to 10 passages were subjected to the experiment.

VSMC were cultured in a medium containing 10% serum for 24 hours and in a serum free medium for 24 hours to synchronize the cell cycle. The VSMC were transferred to a medium containing 10% fetal calf serum or a serum free medium and [³H] thymidine (2 µCi/ml) was added to each medium to label VSMC. The test drug (Y-27632) (10 µM or 30 µM) waw simultaneously added, and after 24 hours of culture, VSMC were recovered, admixed with a scintillator (ACSII, Amersham) and subjected to the measurement of radioactivity by a liquid scintillation counter (LS6500, Beckman), based on which the [³H] thymidine uptake was measured. The results are shown in Table 1.

### (Results)

**Table 1**

| | Vehicle | Y-27632 | |
|---|---|---|---|
| | | 10 µM | 30 µM |
| FCS-free | 143919 ± 8716 | 150381 ± 6515 | 127767 ± 2482 |
| + 10% FCS | 342304 ± 7859 | 360153 ± 10425 | 366089 ± 12882 |
| (dpm/dish, n=5) | | | |

VSMC cultured in a medium containing 10% serum (serum stimulation group) showed an increase of about 230% in the [³H] thymidine uptake, as compared to VSMC cultured in a serum free medium (control group). In contrast, the addition of the test drug (Y-27632) did not affect the [³H] thymidine uptake of neither the serum stimulation group nor the control group.

### (2) Effect on migration ability of cultured VSMC (method)

A serum free medium or a medium containing 10% serum, which contained the test drug (Y-27632, 10 µM), was placed in a 96 well chemotaxis lower chamber, and a serum free medium containing the test drug was placed in 96 well chemotaxis upper and lower chambers where VSMC were suspended, and then cultured for 18 hours. The VSMC migrated to the lower surface of the membrane were stained with QuickDiff (International Reagents Corporation) and absorbance was measured at A595. The results are shown in Table 2.

### (Results)

**Table 2**

| A595 | vehicle | Y-27632 (10 µM) |
|---|---|---|
| FCS-Free | 0.018 ± 0.006 | 0.007 ± 0.003 |
| + 10% FCS | 0.143 ± 0.011 | 0.036 ± 0.010 |
| | | (arbitrary unit/well) |

The test drug (Y-27632) suppressed the migration ability of both VSMC cultured in the medium containing 10% serum and VSMC cultured in the serum free medium.

### Experimental Example 2: Suppressive effect on proliferation of regenerated intima after injury

### Effect on proliferation of regenerated intima after balloon injury of carotid artery in rat

### (Method)

2F Fogarty catheter was inserted from the outer left carotid artery of 8-week-old male WKY rats under anesthesia and inflated in the left common carotid artery, whereby intima was detached in the entire length. Physiological saline was consecutively administered to a control group, and the test drug (Y-27632) (30 mg/kg) was consecutively administered to a test group, wherein both groups underwent intraperitoneal administration starting from 3 days before operation. The rats free of the intima detachment treatment were used as a sham group. At 14 days after the operation, left carotid artery was subjected to perfusion fixation and removed thereafter, stained with HE, and a new intima thickness/medial thickness (I/M) ratio was measured. The left carotid arteries removed and stained with HE were photographed and are shown in Figs. 1-3.

### (Results)

In the control group, proliferation of new intima mainly consisting of VSMC proliferation was observed, with an I/M ratio of 1.88±0.24 significantly increased from the sham group (I/M ratio:0.03±0.01), thus evidencing lumen constriction. In contrast, the Y-27632 administration group showed a significant decrease in the I/M ratio to 0.46±0.22, due to the suppression of neogenesis of intima.

### Industrial Applicability

From the above-mentioned Formulation Examples and Pharmacological Experiments, it is evident that a compound having a Rho kinase inhibitory activity suppresses regenerative intima proliferation after injury of blood vessel and has various other actions. Therefore, it is useful as an agent for the prophylaxis and treatment of vascular constriction, specifically, an agent for the prophylaxis and treatment of vascular constriction induced by disorder of vascular wall, such as vascular restenosis that occurs after an operation of percutaneus transluminal coronary angioplasty, vascular restenosis that occurs after an operation of percutaneus transluminal angioplasty, vascular constriction that occurs after vascular reconstruction, such as DCA, operation of intravascular indwelling of stent and the like, and vascular constriction that occurs after organ transplantation.

This application is based on a patent application No. 114775/1999 filed in Japan, the contents of which are hereby incorporated by reference.

## Claims

1. An agent for the prophylaxis and treatment of vascular constriction, which comprises a compound having a Rho kinase inhibitory activity.

2. The agent for the prophylaxis and treatment of vascular constriction of claim 1, wherein the compound having a Rho kinase inhibitory activity is an amide compound of the following formula (I) 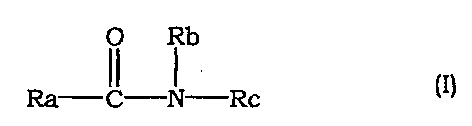 wherein
Ra is a group of the formula 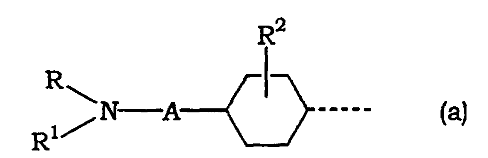 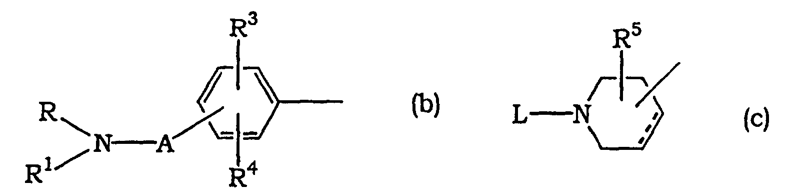 in the formulas *(a)* and *(b),*
R is hydrogen, alkyl, or cycloalkyl, cycloalkylalkyl, phenyl or aralkyl, which optionally has a substituent on the ring, or a group of the formula 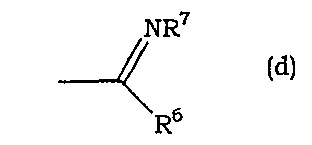 wherein R⁶ is hydrogen, alkyl or formula : -NR⁸R⁹
wherein R⁸ and R⁹ are the same or different and each is hydrogen, alkyl, aralkyl or phenyl, R⁷ is hydrogen, alkyl, aralkyl, phenyl, nitro or cyano,
or R⁶ and R⁷ in combination show a group forming a heterocycle optionally having, in the ring, oxygen atom, sulfur atom or optionally substituted nitrogen atom,
R¹ is hydrogen, alkyl, or cycloalkyl, cycloalkylalkyl, phenyl or aralkyl, which optionally has a substituent on the ring, or
R^{•} and R¹ in combination form, together with the adjacent nitrogen atom, a group forming a heterocycle optionally having, in the ring, oxygen atom, sulfur atom or optionally substituted nitrogen atom,
R² is hydrogen or alkyl,
R³ and R⁴ are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and
A is a group of the formula 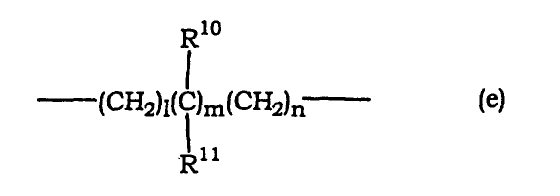 wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
in the formula (c),
L is hydrogen, alkyl, aminoalkyl, mono- or dialkylaminoalkyl, tetrahydrofurfuryl, carbamoylalkyl, phthalimidoalkyl, amidino or a group of the formula 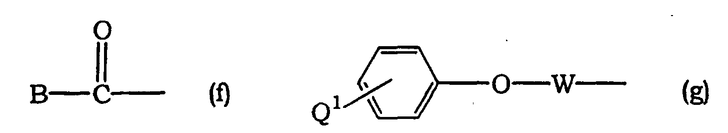 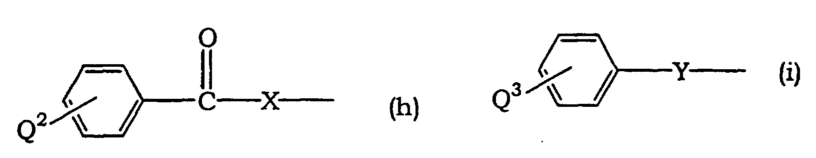 wherein B is hydrogen, alkyl, alkoxy, aralkyl, aralkyloxy, aminoalkyl, hydroxyalkyl, alkanoyloxyalkyl, alkoxycarbonylalkyl, α-aminobenzyl, furyl, pyridyl, phenyl, phenylamino, styryl or imidazopyridyl,
Q¹ is hydrogen, halogen, hydroxy, aralkyloxy or thienylmethyl,
W is alkylene,
Q² is hydrogen, halogen, hydroxy or aralkyloxy,
X is alkylene,
Q³ is hydrogen, halogen, hydroxy, alkoxy, nitro, amino, 2,3-dihydrofuryl or 5-methyl-3-oxo-2,3,4,5-tetrahydropyridazin-6-yl;
and Y is a single bond, alkylene or alkenylene, and
in the formula (c),
a broken line is a single bond or a double bond, and
R⁵ is hydrogen, hydroxy, alkoxy, alkoxycarbonyloxy, alkanoyloxy or aralkyloxycarbonyloxy;
Rb is a hydrogen, an alkyl, an aralkyl, an aminoalkyl or a mono- or dialkylaminoalkyl; and
Rc is an optionally substituted heterocycle containing nitrogen,
an isomer thereof and/or a pharmaceutically acceptable acid addition salt thereof.

3. The agent for the prophylaxis and treatment of vascular constriction of claim 1 or claim 2, wherein the compound having a Rho kinase inhibitory activity is an amide compound of the following formula (I') 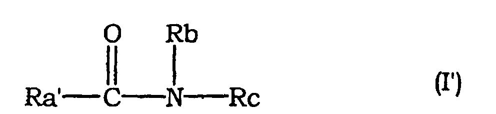 wherein
Ra' is a group of the formula 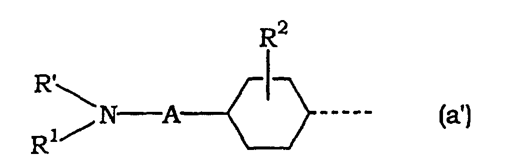 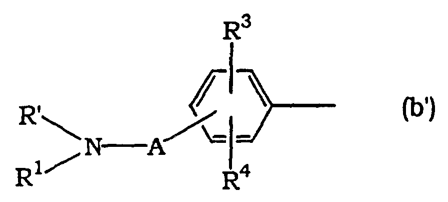
wherein
R' is hydrogen, alkyl, or cycloalkyl, cycloalkylalkyl, phenyl or aralkyl, which optionally has a substituent on the ring,
R¹ is hydrogen, alkyl, or cycloalkyl, cycloalkylalkyl, phenyl or aralkyl, which optionally has a substituent on the ring, or
R' and R¹ in combination form, together with the adjacent nitrogen atom, a group forming a heterocycle optionally having, in the ring, oxygen atom, sulfur atom or optionally substituted nitrogen atom,
R² is hydrogen or alkyl,
R³ and R⁴ are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and
A is a group of the formula 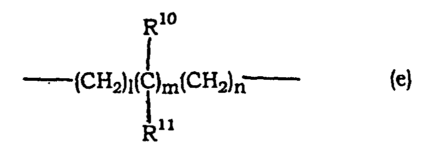 wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
Rb is a hydrogen, an alkyl, an aralkyl, an aminoalkyl or a mono- or dialkylaminoalkyl; and
Rc is an optionally substituted heterocycle containing nitrogen,
an isomer thereof and/or a pharmaceutically acceptable acid addition salt thereof.

4. The agent for the prophylaxis and treatment of vascular constriction of claim 1, wherein the compound having a Rho kinase inhibitory activity is a compound selected from the group consisting of (+)-trans-4-(1-aminoethyl)-1-(4-pyridylcarbamoyl)cyclohexane, (+)-trans-N-(1H-pyrrolo[2,3-b]pyridin-4-yl)-4-(1-aminoethyl)cyclohexanecarboxamide, (R)-(+)-N-(4-pyridyl)-4-(1-aminoethyl)benzamide and (R)-(+)-N-(1H-pyrrolo[2,3-b]pyridin-4-yl)-4-(1-aminoethyl)benzamide, and/or a pharmaceutically acceptable acid addition salt thereof.

5. The agent for the prophylaxis and treatment of vascular constriction of claim 1, wherein the compound having a Rho kinase inhibitory activity is (+)-trans-4-(1-aminoethyl)-1-(4-pyridylcarbamoyl)cyclohexane and/or a pharmaceutically acceptable acid addition salt thereof.

6. The agent for the prophylaxis and treatment of vascular constriction of any of claim 1 to claim 5, wherein the vascular constriction is induced by a disorder of a vascular wall.

7. The agent for the prophylaxis and treatment of vascular constriction of any of claim 1 to claim 5, wherein the vascular constriction is vascular restenosis that occurs after an operation of percutaneus transluminal coronary angioplasty, vascular restenosis that occurs after an operation of percutaneus transluminal angioplasty, vascular constriction that occurs after vascular reconstruction, such as DCA, operation of intravascular indwelling of stent and the like, or vascular constriction that occurs after organ transplantation.

8. A pharmaceutical composition for the prophylaxis and treatment of vascular constriction, which comprises a compound having a Rho kinase inhibitory activity and a pharmaceutically acceptable carrier.

9. The pharmaceutical composition for the prophylaxis and treatment of vascular constriction of claim 8, wherein the compound having a Rho kinase inhibitory activity is an amide compound of the formula (I) 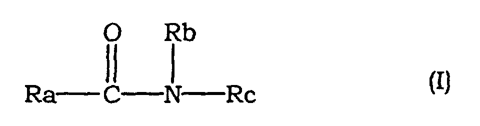 wherein
Ra is a group of the formula 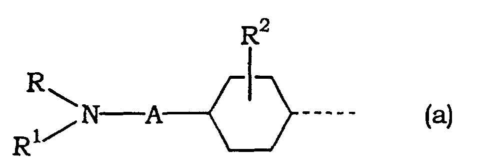 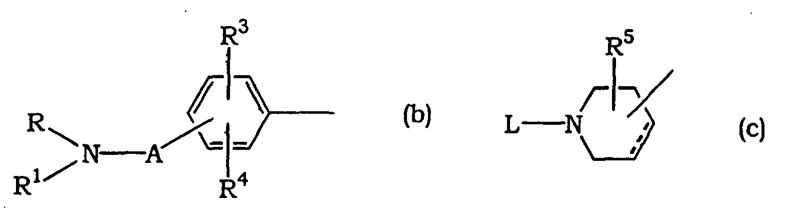
in the formulas (a) and (b),
R is hydrogen, alkyl, or cycloalkyl, cycloalkylalkyl, phenyl or aralkyl, which optionally has a substituent on the ring, or a group of the formula 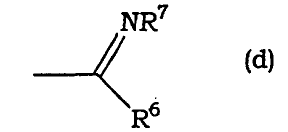 wherein R⁶ is hydrogen, alkyl or formula : -NR⁸R⁹ wherein R⁸ and R⁹ are the same or different and each is hydrogen, alkyl, aralkyl or phenyl, R⁷ is hydrogen, alkyl, aralkyl, phenyl, nitro or cyano, or R⁶ and R⁷ in combination show a group forming a heterocycle optionally having, in the ring, oxygen atom, sulfur atom or optionally substituted nitrogen atom,
R¹ is hydrogen, alkyl, or cycloalkyl, cycloalkylalkyl, phenyl or aralkyl, which optionally has a substituent on the ring, or
R and R¹ in combination form, together with the adjacent nitrogen atom, a group forming a heterocycle optionally having, in the ring, oxygen atom, sulfur atom or optionally substituted nitrogen atom,
R² is hydrogen or alkyl,
R³ and R⁴ are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and
A is a group of the formula 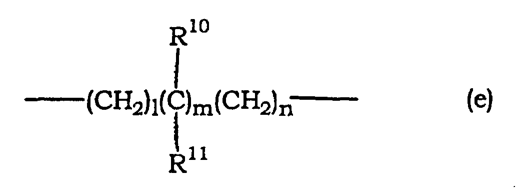 wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
in the formula (c),
L is hydrogen, alkyl, aminoalkyl, mono- or dialkylaminoalkyl, tetrahydrofurfuryl, carbamoylalkyl, phthalimidoalkyl, amidino or a group of the formula 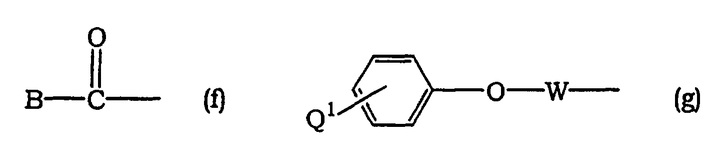 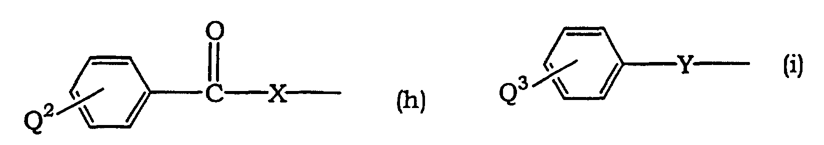 wherein B is hydrogen, alkyl, alkoxy, aralkyl, aralkyloxy, aminoalkyl, hydroxyalkyl, alkanoyloxyalkyl, alkoxycarbonylalkyl, α-aminobenzyl, furyl, pyridyl, phenyl, phenylamino, styryl or imidazopyridyl,
Q¹ is hydrogen, halogen, hydroxy, aralkyloxy or thienylmethyl,
W is alkylene,
Q² is hydrogen, halogen, hydroxy or aralkyloxy,
X is alkylene,
Q³ is hydrogen, halogen, hydroxy, alkoxy, nitro, amino, 2,3-dihydrofuryl or 5-methyl-3-oxo-2,3,4,5-tetrahydropyridazin-6-yl;
and Y is a single bond, alkylene or alkenylene, and
in the formula (c),
a broken line is a single bond or a double bond, and
R⁵ is hydrogen, hydroxy, alkoxy, alkoxycarbonyloxy, alkanoyloxy or aralkyloxycarbonyloxy;
Rb is a hydrogen, an alkyl, an aralkyl, an aminoalkyl or a mono- or dialkylaminoalkyl; and
Rc is an optionally substituted heterocycle containing nitrogen,
an isomer thereof and/or a pharmaceutically acceptable acid addition salt thereof.

10. The pharmaceutical composition for the prophylaxis and treatment of vascular constriction of claim 8 or claim 9, wherein the compound having a Rho kinase inhibitory activity is an amide compound of the formula (I') 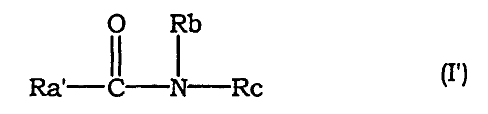 wherein
Ra' is a group of the formula 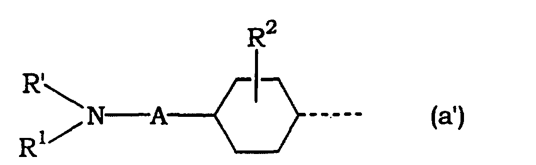 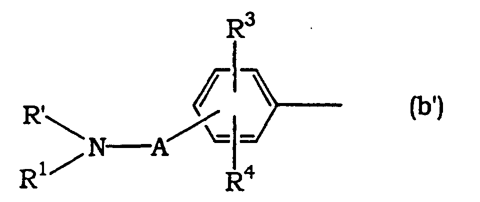
wherein
R' is hydrogen, alkyl, or cycloalkyl, cycloalkylalkyl, phenyl or aralkyl, which optionally has a substituent on the ring,
R¹ is hydrogen, alkyl, or cycloalkyl, cycloalkylalkyl, phenyl or aralkyl, which optionally has a substituent on the ring, or
R' and R¹ in combination form, together with the adjacent nitrogen atom, a group forming a heterocycle optionally having, in the ring, oxygen atom, sulfur atom or optionally substituted nitrogen atom,
R² is hydrogen or alkyl,
R³ and R⁴ are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and
A is a group of the formula 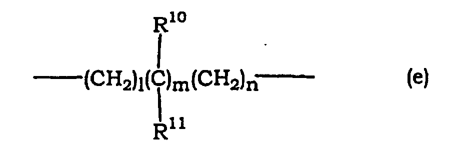 wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
Rb is a hydrogen, an alkyl, an aralkyl, an aminoalkyl or a mono- or dialkylaminoalkyl; and
Rc is an optionally substituted heterocycle containing nitrogen,
an isomer thereof and/or a pharmaceutically acceptable acid addition salt thereof.

11. The pharmaceutical composition for the prophylaxis and treatment of vascular constriction of claim 8, wherein the compound having a Rho kinase inhibitory activity is a compound selected from the group consisting of (+)-trans-4-(1-aminoethyl)-1-(4-pyridylcarbamoyl)cyclohexane, (+)-trans-N-(1H-pyrrolo[2,3-b]pyridin-4-yl)-4-(1-aminoethyl)cyclohexanecarboxamide, (R)-(+)-N-(4-pyridyl)-4-(1-aminoethyl)benzamide and (R)-(+)-N-(1H-pyrrolo[2,3-b]pyridin-4-yl)-4-(1-aminoethyl)benzamide, and/or a pharmaceutically acceptable acid addition salt thereof.

12. The pharmaceutical composition for the prophylaxis and treatment of vascular constriction of claim 8, wherein the compound having a Rho kinase inhibitory activity is (+)-trans-4-(1-aminoethyl)-1-(4-pyridylcarbamoyl)cyclohexane and/or a pharmaceutically acceptable acid addition salt thereof.

13. The pharmaceutical composition for the prophylaxis and treatment of vascular constriction of any of claim 8 to claim 12, wherein the vascular constriction is induced by a disorder of a vascular wall.

14. The pharmaceutical composition for the prophylaxis and treatment of vascular constriction of any of claim 8 to claim 12, wherein the vascular constriction is vascular restenosis that occurs after an operation of percutaneus transluminal coronary angioplasty, vascular restenosis that occurs after an operation of percutaneus transluminal angioplasty, vascular constriction that occurs after vascular reconstruction, such as DCA, operation of intravascular indwelling of stent and the like, or vascular constriction that occurs after organ transplantation.

15. A method of the prophylaxis and treatment of vascular constriction, which comprises administering an effective amount of a compound having a Rho kinase inhibitory activity to a patient.

16. The method of the prophylaxis and treatment of vascular constriction of claim 15, wherein the compound having a Rho kinase inhibitory activity is an amide compound of the formula (I) 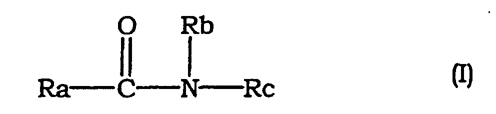 wherein
Ra is a group of the formula 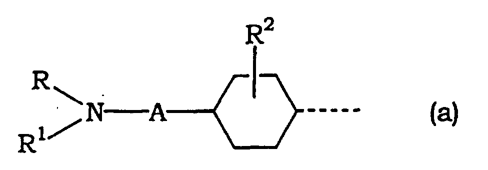 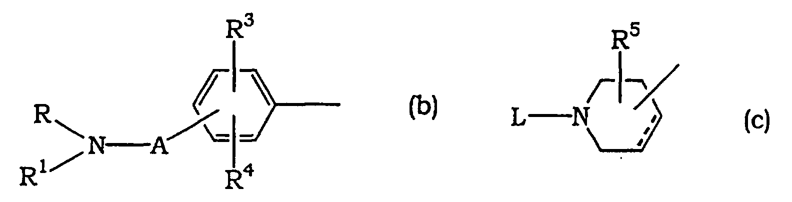
in the formulas (a) and (b),
R is hydrogen, alkyl, or cycloalkyl, cycloalkylalkyl, phenyl or aralkyl, which optionally has a substituent on the ring, or a group of the formula 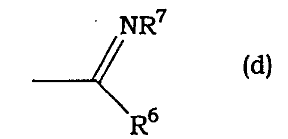 wherein R⁶ is hydrogen, alkyl or formula : -NR⁸R⁹
wherein R⁸ and R⁹ are the same or different and each is hydrogen, alkyl, aralkyl or phenyl, R⁷ is hydrogen, alkyl, aralkyl, phenyl, nitro or cyano, or R⁶ and R⁷ in combination show a group forming a heterocycle optionally having, in the ring, oxygen atom, sulfur atom or optionally substituted nitrogen atom,
R¹ is hydrogen, alkyl, or cycloalkyl, cycloalkylalkyl, phenyl or aralkyl, which optionally has a substituent on the ring, or
R and R¹ in combination form, together with the adjacent nitrogen atom, a group forming a heterocycle optionally having, in the ring, oxygen atom, sulfur atom or optionally substituted nitrogen atom,
R² is hydrogen or alkyl,
R³ and R⁴ are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and
A is a group of the formula 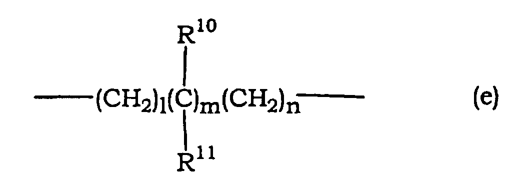 wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
in the formula (c),
L is hydrogen, alkyl, aminoalkyl, mono- or dialkylaminoalkyl, tetrahydrofurfuryl, carbamoylalkyl, phthalimidoalkyl, amidino or a group of the formula 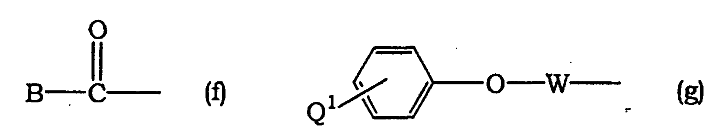 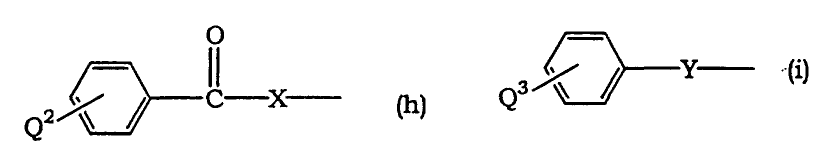 wherein B is hydrogen, alkyl, alkoxy, aralkyl, aralkyloxy, aminoalkyl, hydroxyalkyl, alkanoyloxyalkyl, alkoxycarbonylalkyl, α-aminobenzyl, furyl, pyridyl, phenyl, phenylamino, styryl or imidazopyridyl,
Q¹ is hydrogen, halogen, hydroxy, aralkyloxy or thienylmethyl,
W is alkylene,
Q² is hydrogen, halogen, hydroxy or aralkyloxy,
X is alkylene,
Q³ is hydrogen, halogen, hydroxy, alkoxy, nitro, amino, 2,3-dihydrofuryl or 5-methyl-3-oxo-2,3,4,5-tetrahydropyridazin-6-yl;
and Y is a single bond, alkylene or alkenylene, and in the formula (c),
a broken line is a single bond or a double bond, and
R⁵ is hydrogen, hydroxy, alkoxy, alkoxycarbonyloxy, alkanoyloxy or aralkyloxycarbonyloxy;
Rb is a hydrogen, an alkyl, an aralkyl, an aminoalkyl or a mono- or dialkylaminoalkyl; and
Rc is an optionally substituted heterocycle containing nitrogen,
an isomer thereof and/or a pharmaceutically acceptable acid addition salt thereof.

17. The method of the prophylaxis and treatment of vascular constriction of claim 15 or claim 16, wherein the compound having a Rho kinase inhibitory activity is an amide compound of the formula (I') 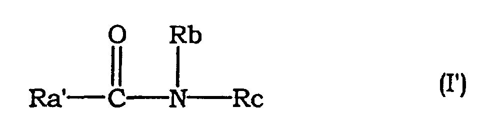 wherein
Ra' is a group of the formula 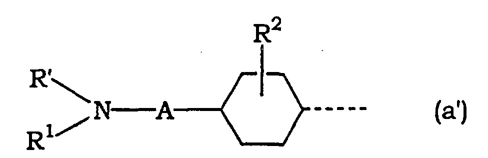 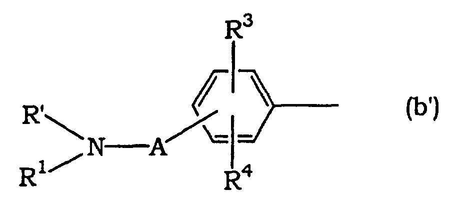
wherein
R' is hydrogen, alkyl, or cycloalkyl, cycloalkylalkyl, phenyl or aralkyl, which optionally has a substituent on the ring,
R¹ is hydrogen, alkyl, or cycloalkyl, cycloalkylalkyl, phenyl or aralkyl, which optionally has a substituent on the ring, or
R' and R¹ in combination form, together with the adjacent nitrogen atom, a group forming a heterocycle optionally having, in the ring, oxygen atom, sulfur atom or optionally substituted nitrogen atom,
R² is hydrogen or alkyl,
R³ and R⁴ are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and
A is a group of the formula 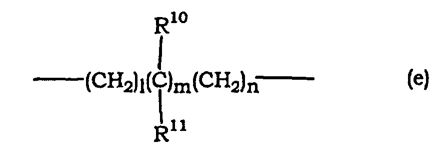 wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
Rb is a hydrogen, an alkyl, an aralkyl, an aminoalkyl or a mono- or dialkylaminoalkyl; and
Rc is an optionally substituted heterocycle containing nitrogen,
an isomer thereof and/or a pharmaceutically acceptable acid addition salt thereof.

18. The method of the prophylaxis and treatment of vascular constriction of claim 15, wherein the compound having a Rho kinase inhibitory activity is a compound selected from the group consisting of (+)-trans-4-(1-aminoethyl)-1-(4-pyridylcarbamoyl)cyclohexane, (+)-trans-N-(1H-pyrrolo[2,3-b]pyridin-4-yl)-4-(1-aminoethyl)cyclohexanecarboxamide, (R)-(+)-N-(4-pyridyl)-4-(1-aminoethyl)benzamide and (R)-(+)-N-(1H-pyrrolo[2,3-b]pyridin-4-yl)-4-(1-aminoethyl)benzamide, and/or a pharmaceutically acceptable acid addition salt thereof.

19. The method of the prophylaxis and treatment of vascular constriction of claim 15, wherein the compound having a Rho kinase inhibitory activity is a (+)-trans-4-(1-aminoethyl)-1-(4-pyridylcarbamoyl)cyclohexane, and/or a pharmaceutically acceptable acid addition salt thereof.

20. The method for the prophylaxis or treatment of vascular constriction of any of claim 15 to claim 19, wherein the vascular constriction is induced by a disorder of a vascular wall.

21. The method for the prophylaxis or treatment of vascular constriction of any of claim 15 to claim 19, wherein the vascular constriction is vascular restenosis that occurs after an operation of percutaneus transluminal coronary angioplasty, vascular restenosis that occurs after an operation of percutaneus transluminal angioplasty, vascular constriction that occurs after vascular reconstruction, such as DCA, operation of intravascular indwelling of stent and the like, or vascular constriction that occurs after organ transplantation.

22. Use of a compound having a Rho kinase inhibitory activity for the production of an agent for the prophylaxis and treatment of vascular constriction.

23. The use of claim 22, wherein the compound having a Rho kinase inhibitory activity is an amide compound of the following formula (I) 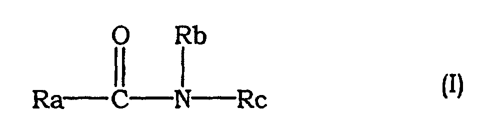 wherein
Ra is a group of the formula 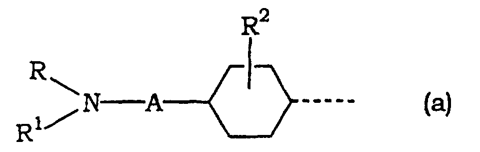 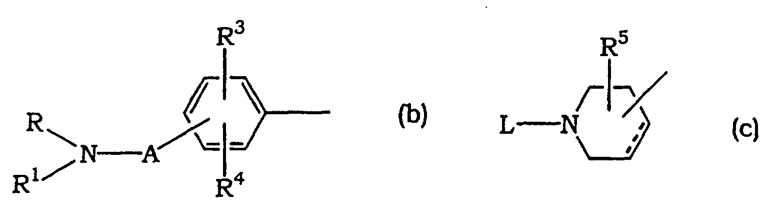
in the formulas (a) and (b),
R is hydrogen, alkyl, or cycloalkyl, cycloalkylalkyl, phenyl or aralkyl, which optionally has a substituent on the ring, or a group of the formula 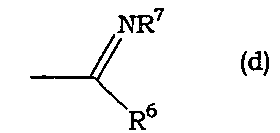 wherein R⁶ is hydrogen, alkyl or formula : -NR⁸R⁹
wherein R⁸ and R⁹ are the same or different and each is hydrogen, alkyl, aralkyl or phenyl, R⁷ is hydrogen, alkyl, aralkyl, phenyl, nitro or cyano, or R⁶ and R⁷ in combination show a group forming a heterocycle optionally having, in the ring, oxygen atom, sulfur atom or optionally substituted nitrogen atom,
R¹ is hydrogen, alkyl, or cycloalkyl, cycloalkylalkyl, phenyl or aralkyl, which optionally has a substituent on the ring, or
R and R¹ in combination form, together with the adjacent nitrogen atom, a group forming a heterocycle optionally having, in the ring, oxygen atom, sulfur atom or optionally substituted nitrogen atom,
R² is hydrogen or alkyl,
R³ and R⁴ are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and
A is a group of the formula 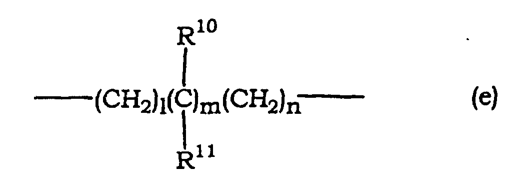 wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
in the formula (c),
L is hydrogen, alkyl, aminoalkyl, mono- or dialkylaminoalkyl, tetrahydrofurfuryl, carbamoylalkyl, phthalimidoalkyl, amidino or a group of the formula 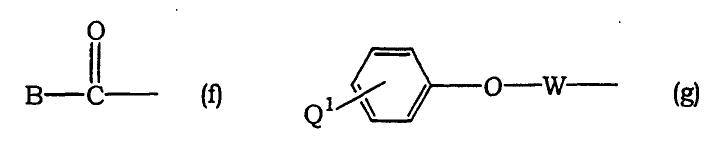 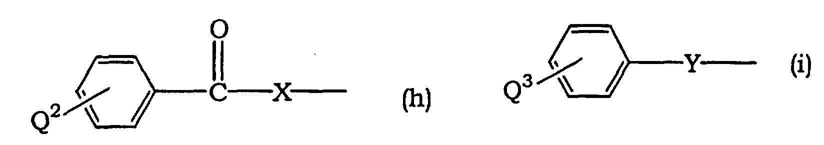 wherein B is hydrogen, alkyl, alkoxy, aralkyl, aralkyloxy, aminoalkyl, hydroxyalkyl, alkanoyloxyalkyl, alkoxycarbonylalkyl, α-aminobenzyl, furyl, pyridyl, phenyl, phenylamino, styryl or imidazopyridyl,
Q¹ is hydrogen, halogen, hydroxy, aralkyloxy or thienylmethyl,
W is alkylene,
Q² is hydrogen, halogen, hydroxy or aralkyloxy,
X is alkylene,
Q³ is hydrogen, halogen, hydroxy, alkoxy, nitro, amino, 2,3-dihydrofuryl or 5-methyl-3-oxo-2,3,4,5-tetrahydropyridazin-6-yl;
and Y is a single bond, alkylene or alkenylene, and
in the formula (c),
a broken line is a single bond or a double bond, and
R⁵ is hydrogen, hydroxy, alkoxy, alkoxycarbonyloxy, alkanoyloxy or aralkyloxycarbonyloxy;
Rb is a hydrogen, an alkyl, an aralkyl, an aminoalkyl or a mono- or dialkylaminoalkyl; and
Rc is an optionally substituted heterocycle containing nitrogen,
an isomer thereof and/or a pharmaceutically acceptable acid addition salt thereof.

24. The use of claim 22 or claim 23, wherein the compound having a Rho kinase inhibitory activity is an amide compound of the following formula (I') 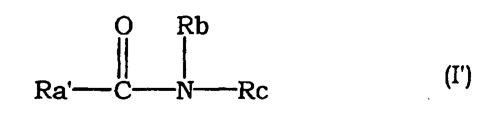 wherein
Ra' is a group of the formula 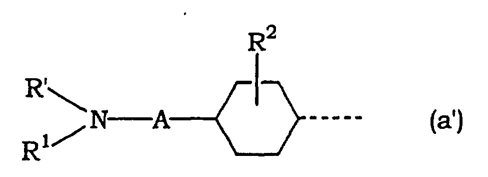 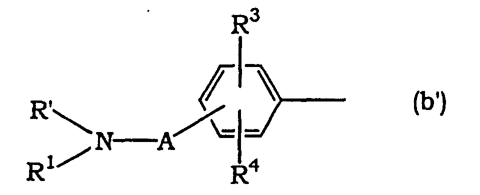
wherein
R' is hydrogen, alkyl, or cycloalkyl, cycloalkylalkyl, phenyl or aralkyl, which optionally has a substituent on the ring,
R¹ is hydrogen, alkyl, or cycloalkyl, cycloalkylalkyl, phenyl or aralkyl, which optionally has a substituent on the ring, or
R' and R¹ in combination form, together with the adjacent nitrogen atom, a group forming a heterocycle optionally having, in the ring, oxygen atom, sulfur atom or optionally substituted nitrogen atom,
R² is hydrogen or alkyl,
R³ and R⁴ are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and
A is a group of the formula 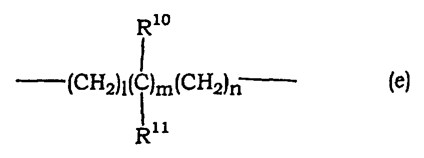 wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
Rb is a hydrogen, an alkyl, an aralkyl, an aminoalkyl or a mono- or dialkylaminoalkyl; and
Rc is an optionally substituted heterocycle containing nitrogen,
an isomer thereof and/or a pharmaceutically acceptable acid addition salt thereof.

25. The use of claim 22, wherein the compound having a Rho kinase inhibitory activity is a compound selected from the group consisting of (+)-trans-4-(1-aminoethyl)-1-(4-pyridylcarbamoyl)cyclohexane, (+)-trans-N-(1H-pyrrolo[2,3-b]pyridin-4-yl)-4-(1-aminoethyl)cyclohexanecarboxamide, (R)-(+)-N-(4-pyridyl)-4-(1-aminoethyl)benzamide and (R)-(+)-N-(1H-pyrrolo[2,3-b]pyridin-4-yl)-4-(1-aminoethyl)benzamide, and/or a pharmaceutically acceptable acid addition salt thereof.

26. The use of claim 22, wherein the compound having a Rho kinase inhibitory activity is a (+)-trans-4-(1-aminoethyl)-1-(4-pyridylcarbamoyl)cyclohexane, and/or a pharmaceutically acceptable acid addition salt thereof.

27. The use of any of claim 22 to claim 26, wherein the vascular constriction is induced by a disorder of a vascular wall.

28. The use of any of claim 22 to claim 26, wherein the vascular constriction is vascular restenosis that occurs after an operation of percutaneus transluminal coronary angioplasty, vascular restenosis that occurs after an operation of percutaneus transluminal angioplasty, vascular constriction that occurs after vascular reconstruction, such as DCA, operation of intravascular indwelling of stent and the like, or vascular constriction that occurs after organ transplantation.

29. A commercial package comprising a pharmaceutical composition for the prophylaxis and treatment of vascular constriction of any of claim 8 to claim 14, and a written matter associated therewith, the written matter stating that the pharmaceutical composition can or should be used for the prophylaxis and treatment of vascular constriction.
